# EUROPEAN PATENT SPECIFICATION

(11) **EP 0 529 715 B1**
(45) Date of publication and mention of the grant of the patent: **05.03.1997**
(21) Application number: 92202507.7
(22) Date of filing: 17.08.1992
(51) Int. Cl.: C07H 11/00, C07H 15/04, C07H 15/26, C07H 15/08, A61K 31/70

(54) **Sulfated glycosaminoglycanoid derivatives**
Sulfatierte Glykosaminoglycanoidderivate
Dérivés sulfatés de glycosaminoglycanoides

(30) Priority: 23.08.1991 EP 91402299
(43) Date of publication of application: 03.03.1993
(73) Proprietor: Akzo Nobel N.V., 6824 BM Arnhem (NL); ELF SANOFI, 75008 Paris (FR)
(72) Inventor: Petitou, Maurice, F-75013 Paris (FR); van Boeckel, Constant Adriaan Anton, NL-5345 LX Oss (NL)
(74) Representative: Hermans, Franciscus G.M.

(56) References cited:
- EP-A- 0 230 023
- EP-A- 0 300 099
- EP-A- 0 301 618
- EP-A- 0 454 220
- GB-A- 1 110 939
- US-A- 3 017 407

## Description

The invention concerns a sulfated glycosaminoglycan derivative or a pharmaceutically acceptable salt thereof, wherein all functional N-sulfate, N-acetate, and hydroxy groups, are replaced by alkoxy, aryloxy, aralkoxy, O-sulfate, or OALK(OALK)ₙOX groups, wherein ALK is an aliphatic hydrocarbon group, n is 0-5, and X is an alkyl or aryl group, provided that at least two functional groups are not replaced by O-sulfate. The invention is further related to a process for the preparation of said derivative, a pharmaceutical composition containing the same, as well as to a use of sulfated glycosaminoglycan derivatives for the preparation of a medicament.

In some contracting states a European patent application was filed (EP 454,220) disclosing glycosaminoglycan derivatives comprising the trisaccharide unit having the structure β-D-glucopyranuronosyl-(1→4)-O-2,3,6-tri-O-sulfo-α-D-glucopyranosyl-(1→4)-O-2-O-sulfo-α-L-idopyranuronosyl and alkylated, arylated, aralkylated, or sulfated derivatives thereof. In these countries protection is sought for a sulfated glycosaminoglycan derivative or a pharmaceutically acceptable salt thereof, wherein all functional N-sulfate, N-acetate, and hydroxy groups, are replaced by alkoxy, aryloxy, aralkoxy, O-sulfate, or OALK(OALK)ₙOX groups, wherein ALK is an aliphatic hydrocarbon group, n is 0-5, and X is an alkyl or aryl group, with the proviso that glycosaminoglycan derivatives comprising the trisaccharide unit having the structure β-D-glucopyranuronosyl-(1→4)-O-2,3,6-tri-O-sulfo-α-D-glucopyranosyl-(1→4)-O-2-O-sulfo-α-L-idopyranuronosyl and alkylated, arylated, aralkylated, or sulfated derivatives thereof, are excluded, provided that at least two functional groups are not replaced by O-sulfate.

Sulfated glycosaminoglycan derivatives are known. European patent EP 84,999, for instance, discloses sulfated pentasaccharides of the chemical class of glycosaminoglycans having antithrombotic activity. These known compounds can possess, apart from hydroxy groups, O-sulfate, N-sulfate, and N-acetyl groups, whereas the anomeric hydroxy group is sometimes replaced by a methoxy group. When all functional groups are replaced by O-sulfate, persulfated carbohydrates such as maltose octasulfate as disclosed in EP 230,023 are obtained, but such compounds normally not longer have the desired antithrombotic activity, and therefore they are not encompassed in this invention. Also glycosaminoglycan derivatives having a non-sulfated anomeric hydroxy group but all other functional groups being replaced by O-sulfate, do not longer have the desired antithrombotic activity. Apart from the anomeric hydroxy group at least one other functional group should be alkoxy, aryloxy, aralkoxy, or OALK(OALK)ₙOX, and preferably alkoxy.

In contrast to the known glycosaminoglycan derivatives, the present sulfated glycosaminoglycan derivatives do not have free hydroxy groups, nor do they possess N-sulfate or N-acetyl groups.

It has now been found that the compounds of this invention have a better binding affinity to antithrombin III with respect to the naturally occuring pentasaccharide of European patent EP 84,999, which results in a better pharmacokinetic profile, longer half-life times, and lower therapeutic doses and thus lesser side-effects. Furthermore, the compounds of this invention have a substantially better heparin cofactor II (HCII) mediated antithrombin activity, and are, therefore, more effective as thrombin generation inhibitors than the prior art compounds. The sulfated glycosaminoglycan derivatives can also be used as inhibitors for smooth muscle cell proliferation, and for the treatment of angiogenesis, cancer, and retrovirus infections, like HIV.
The inclusion of alkyl, aryl, or aralkyl functionalized saccharide units gives further a very important synthetic advantage over the prior art compounds. By functionalizing the hydroxy groups with alkyl, aryl, or aralkyl groups, it is in most cases redundant to prepare temporarily protected carbohydrates, which makes the synthetic pathway considerably shorter and simpler, whereas the replacement of the glucosamine units by glucose units further simplifies the synthesis of the saccharides significantly. Moreover, an additional advantage of the synthesis of the compounds of the invention is that the nature of the temporarily protective groups, which are necessary for the protection of the hydroxy groups to be sulfated, is not critical.

Preferred compounds according to this invention are sulfated glycosaminoglycan derivatives, comprising the disaccharide unit having the formula I 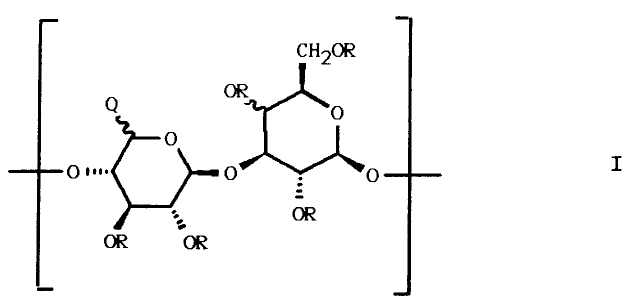 in which the twitched lines denote an α or β bond, each of the groups R are independently selected from the group consisting of alkyl, aryl, aralkyl, and sulfate; and Q is carboxylate or a pharmaceutically acceptable salt thereof.
Other preferred compounds are the sulfated glycosaminoglycan derivatives comprising the disaccharide unit having the formula IIa or IIb 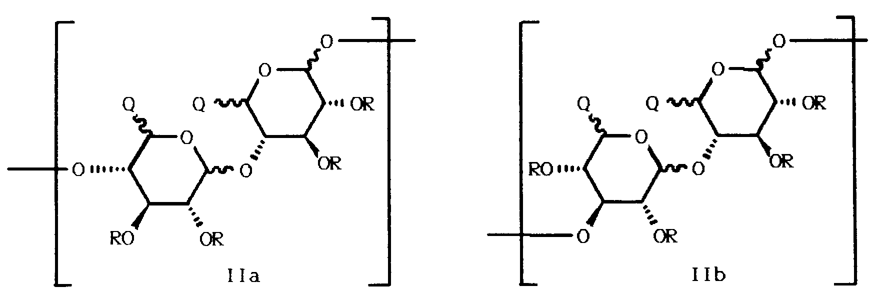 in which the twitched lines denote an α or β bond, each of the groups R are independently selected from the group consisting of alkyl, aryl, aralkyl, and sulfate, and Q is carboxylate, or a pharmaceutically acceptable salt thereof.

Particularly useful compounds according to this invention are the sulfated glycosaminoglycan derivatives having the formula III 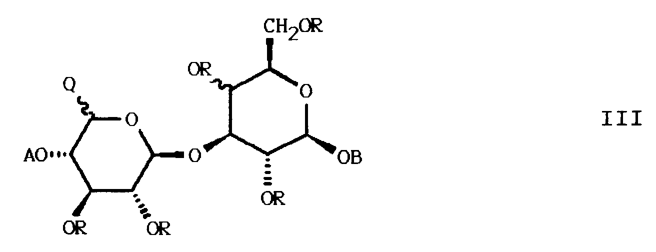 in which the twitched lines denote an α or β bond, each of the groups R are independently selected from the group consisting of alkyl, aryl, aralkyl, and sulfate;
Q is carboxylate;
A is selected from the group consisting of alkyl, aryl, aralkyl, sulfate, and a carbohydrate group having the formula IV 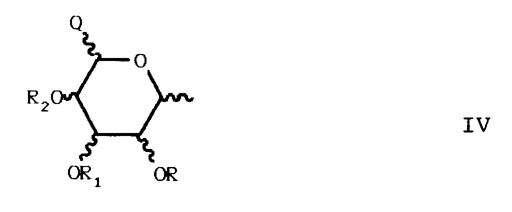 in which the twitched lines, Q, and R have the previously given meanings, one of the groups R₁ and R₂ is alkyl, aryl, aralkyl, or sulfate, whereas the other is selected from the group consisting of alkyl, aryl, aralkyl, sulfate, and a carbohydrate group having the formula V 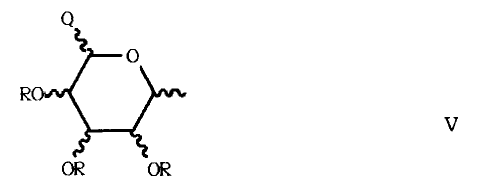 in which the twitched lines, Q, and R have the previously given meanings; and
B is selected from the group consisting of alkyl, aryl, aralkyl, sulfate, ALK(OALK)ₙOX (wherein ALK is an aliphatic hydrocarbon group, n is 0-5, and X is an alkyl or aryl group), and a carbohydrate group having the formula VI 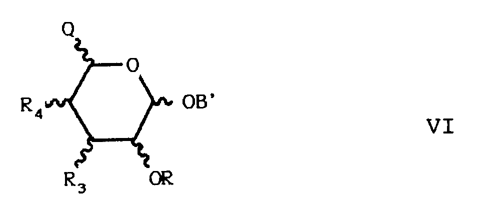 in which the twitched lines, Q, and R have the previously given meanings, one of the groups R₃ and R₄ is alkoxy, aryloxy, aralkoxy, or O-sulfate, whereas the other is a bond, and B' is selected from the group consisting of alkyl, aryl, aralkyl, sulfate, ALK(OALK)ₙOX (wherein ALK, n, and X have the previously given meanings), and a carbohydrate group having the formula VII 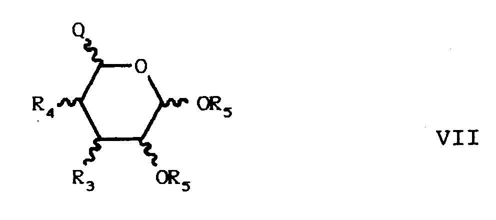 in which the twitched lines, Q, R₃, and R₄ have the previously given meanings, and each of R₅ has independently the meaning previously given for R or is ALK(OALK)ₙOX (wherein ALK, n, and X have the previously given meanings), or a pharmaceutically acceptable salt thereof.

Also useful are the sulfated glycosaminoglycan derivatives having the formula VIII 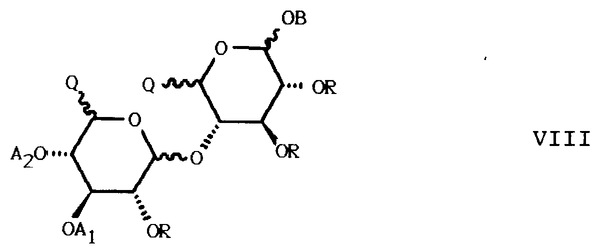 in which the twitched lines denote an α or β bond, each of the groups R are independently selected from the group consisting of alkyl, aryl, aralkyl, and sulfate, Q is carboxylate, one of the groups A₁ and A₂ is alkyl, aryl, aralkyl, or sulfate, whereas the other is selected from the group consisting of alkyl, aryl, aralkyl, sulfate, and a carbohydrate group having the formula IV 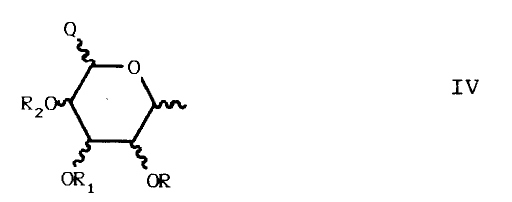 in which the twitched lines, Q, and R have the previously given meanings, one of the groups R₁ and R₂ is alkyl, aryl, aralkyl, or sulfate, whereas the other is selected from the group consisting of alkyl, aryl, aralkyl, sulfate, and a carbohydrate group having the formula V 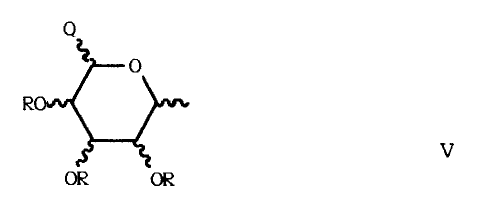 in which the twitched lines, Q, and R have the previously given meanings; and
B is selected from the group consisting of alkyl, aryl, aralkyl, sulfate, ALK(OALK)ₙOX, wherein ALK is an aliphatic hydrocarbon group, n is 0-5, and X is an alkyl or aryl group, and a carbohydrate group having the formula VI 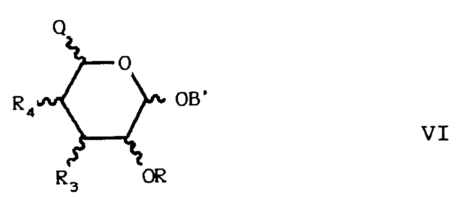 in which the twitched lines, Q, and R have the previously given meanings, one of the groups R₃ and R₄ is alkoxy, aryloxy, aralkoxy, or O-sulfate, whereas the other is a bond, and B' is selected from alkyl, aryl, aralkyl, sulfate, ALK(OALK)ₙOX (wherein ALK, n, and X have the previously given meanings), and a carbohydrate group having the formula VII 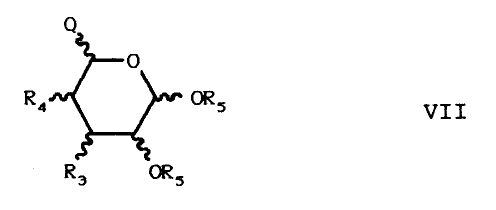 in which the twitched lines, Q, R₃, and R₄ have the previously given meanings, and each of R₅ has independently the meaning previously given for R or is ALK(OALK)ₙOX (wherein ALK, n, and X have the previously given meanings), or a pharmaceutically acceptable salt thereof.

The alkyl group in the definition of R, R₁, R₂, A, A₁, A₂, X, B and B' is an unsubstituted or NR'R''-substituted, branched or unbranched alkyl group having 1-20 carbon atoms or a cyclo-alkyl group having 3-8 carbon atoms. Alkyl groups for different groups R may be different. Examples are methyl, ethyl, isopropyl, butyl, *sec*-butyl, pentyl, neopentyl, hexyl, octyl, decyl, undecyl, dodecyl, tridecyl, tetradecyl, pentadecyl, hexadecyl, octadecyl, and eicosyl. Preferred are the alkyl groups having 1-6 carbon atoms. More preferred are the alkyl groups having 1-4 carbon atoms, and most preferred is a methyl group.
The group NR'R'' is an amino group wherein R' and R'' are independently selected from the group of hydrogen, alkyl (as previously defined), acyl (preferentially acyl groups derived from aliphatic hydrocarbon groups having 2-6 carbon atoms), benzyloxycarbonyl, carboxyl, and SO₃⁻, or R' and R'' form together with the nitrogen atom to which they are bonded a cyclic amide or imide (examples are the phthaloylamino, succinylamino, trimellitylamino and similar groups).

The term aryl in the definition of R, R₁, R₂, A, A₁, A₂, X, B and B' means an aromatic group, preferably phenyl, which may be substituted by OH, alkyl having 1-4 carbon atoms, alkoxy having 1-4 carbon atoms, halogen (preferably fluorine, chlorine, or bromine), CF₃, or NR'R'', wherein R' and R'' have the previously given meanings. The term aralkyl means an aralkyl group in which the alkyl moiety is an alkyl group having 1-4 carbon atoms and the aryl moiety is an aryl group as previously defined.

In the terms alkoxy, aryloxy, and aralkoxy, used in the definitions of R₃ and R₄, the alkyl, aryl, and aralkyl moieties have the same meanings as given previously for the alkyl, aryl, and aralkyl groups respectively in the definition of R, R₁, R₂, A, A₁, A₂, X, B and B'.

The term ALK means an aliphatic hydrocarbon group having 2-6 carbon atoms. ALK can be a branched or unbranched, saturated or unsaturated hydrocarbon group. Preferred ALK groups are saturated, and with more preference unbranched hydrocarbon groups. Preferred ALK groups have 2-4 carbon atoms. Examples of ALK groups are 1,2-ethanediyl, 1,3-propanediyl, 1,4-butanediyl, 1-methyl-1,2-ethanediyl, 2-propene-1,3-diyl, and 2,4-dimethyl-1,4-butanediyl. Most preferred is the 1,2-ethanediyl group.

The subscript "n" in ALK(OALK)ₙOX is an integer between 0 and 5, and preferably between 1 and 3. Most preferred n is 1.

The term "α or β bond" means that the configuration of the concerned bond is respectively trans or cis with respect to the anomeric bond in the concerned saccharide unit.

The sulfated glycosaminoglycan derivative according to the invention means a sulfated glycosaminoglycan derivative, in which the N-sulfate group(s) is (are) replaced by alkoxy, aryloxy, aralkoxy, and preferably by O-sulfate groups, whereas one or more of the uronic acid units may be replaced by carbohydrate units having the group CH₂OR instead of a carboxylate group. A glycosaminoglycan is a carbohydrate which belongs to the well-known chemical class of glycosaminoglycans.

Preferred compounds have R is alkyl, and more preferably methyl, at sites where the corresponding natural occurring glycosaminoglycans possess a free hydroxy group or an acetamido group, and R is sulfate at sites where the corresponding natural occurring glycosaminoglycans possess a sulfate group. Compounds having 5-8 saccharide units (penta to octa-saccharides) are particularly preferred.

It is generally believed that multipoint key polar interactions are of essential importance throughout molecular biology for ensuring high selectivity in non-covalent molecular associations, and that substitution of only one of the key hydroxy groups of an oligosaccharide by a hydrophobic group (and invariably a number of the hydroxy groups prove outstandingly essential to complex formation) can result in complete loss of the affinity by the protein. Remarkably, however, the preferred compounds of this invention having O-alkyl and O-sulfate groups without having free hydroxy groups, still show the full-blown activity.

The counter-ions which compensate the charged moieties are pharmaceutically acceptable counter-ions, like hydrogen, or more preferably alkali or earth-alkali metal ions, like sodium, calcium, or magnesium.

The carbohydrates according to this invention may be prepared according to well known methods described and used for the synthesis of polysaccharides. In this respect, particular reference is made to the previously mentioned European patent EP 84,999, in which methods for the synthesis of polysaccharides are disclosed. A suitable process for the preparation of the sulfated glycosaminoglycan derivative of this invention is characterized in that protected monosaccharides are coupled to give protected disaccharides, which are optionally further coupled to tri- to hexasaccharides, after which the protective groups are partially or completely cleaved and free hydroxy groups are sulfated, after which, if present, remaining protective groups are cleaved, and the compound obtained is optionally converted into a pharmaceutically acceptable salt.

A stepwise condensation of the monosaccharides is possible. In general, however, building blocks consisting of D-glucose, L-idose, D-glucuronic acid or L-iduronic acid, suitably functionalized with the required alkyl, aryl, or aralkyl groups or by temporarily protective groups, are condensed together in the desired order. In this way the (protected) saccharide unit can be prepared, which can be coupled with other saccharide units, or protected derivatives thereof. Suitable protective groups are well known in carbohydrate chemistry. Preferred protective groups include benzyl and acetyl for hydroxy groups, and methyl and benzyl for the carboxylate group of uronic acids. Other protective groups like levulinoyl, chloroacetyl, trityl, benzoyl, and the like, may be used with equal success. Coupling of the saccharides is performed in a manner known in the art, e.g. deprotection of the 1-position of the glycosyl-donor, and/or activation of this position (e.g. by making a bromide, pentenyl, fluoride, thioglycoside, or trichloroacetimide derivative) and coupling the activated glycosyl-donor with an optionally protected glycosyl-acceptor.

For the treatment of venous thrombosis or for the inhibition of smooth muscle cell proliferation the compounds of the invention may be administered enterally or parenterally, and for humans preferably in a daily dosage of 0,001-10 mg per kg body weight. Mixed with pharmaceutically suitable auxiliaries, the compounds may be compressed into solid dosage units, such as pills, tablets, or be processed into capsules or suppositories. By means of pharmaceutically suitable liquids the compounds can also be applied as an injection preparation in the form of a solution, suspension, emulsion, or as a spray, e.g. a nasal spray.

The invention is further illustrated by the following examples.

### Example 1

### 2-(phenylmethyloxycarbonylamino)ethyl O-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo-α-D-glucopyranoside hexakis sodium salt.

a. 2-(phenylmethyloxycarbonylamino)ethyl O-6-O-acetyl-2-O-phenylmethy 3,4-di-O-methyl-α-D-glucopyranosyl-(1→4)-O-(methyl 2,3-di-O-methyl-β-D-glucopyranuronate)-(1→4)-O-3,6-di-O-acetyl-2-O-phenylmethyl-α-D-glucopyranoside (0,0466 mmol) was saponified in the presence of sodium hydroxide to give 2-(phenylmethyloxycarbonylamino)ethyl O-2-O-phenylmethyl-3,4-di-O-methyl-α-D-glucopyranosyl-(1→4)-O-2,3-di-O-methyl-β-D-glucopyranuronosyl-(1→4)-O-2-O-phenylmethyl-α-D-glucopyranoside (57 mg), which was used without further purification.
b. 2-(phenylmethyloxycarbonylamino)ethyl O-2-O-phenylmethyl-3,4-di-O-methyl-α-D-glucopyranosyl-(1→4)-O-2,3-di-O-methyl-β-D-glucopyranuronosyl-(1→4)-O-2-O-phenylmethyl-α-D-glucopyranoside (25 mg) was stirred in *tert*-butanol under an atmosphere of hydrogen for one day in the presence of 10% Pd/C catalyst. After filtration crude 2-aminoethyl O-3,4-di-O-methyl-α-D-glucopyranosyl-(1→4)-O-2,3-di-O-methyl-β-D-glucopyranuronosyl-(1→4)-O-α-D-glucopyranoside was obtained and used as such in the next step.
c. 2-aminoethyl O-3,4-di-O-methyl-α-D-glucopyranosyl-(1→4)-O-2,3-di-O-methyl-β-D-glucopyranuronosyl-(1→4)-O-α-D-glucopyranoside (0,154 mmol) and phenylmethyloxycarbonyl chloride (0,23 mmol) were allowed to react in water (4 ml) in the presence of sodium hydrogen carbonate (35 mg). After gel filtration and evaporation of the solvent, the product (130 mg) was directly used in the sulfation step.
d. 2-(phenylmethyloxycarbonylamino)ethyl O-3,4-di-O-methyl-α-D-glucopyranosyl-(1→4)-O-2,3-di-O-methyl-β-D-glucopyranuronosyl-(1→4)-O-α-D-glucopyranoside (0,154 mmol) and triethylamine sulfur trioxide complex (0,7 g) were allowed to react in N,N-dimethylformamide for 24 h at 50 °C. Sodium hydrogen carbonate (1,2 g) and water (12 ml) were added and after 2 h the mixture was layered on top of a Sephadex G25 column and eluted with water. The fractions were combined and after lyophilisation 2-(phenylmethyloxycarbonylamino)ethyl O-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo-α-D-glucopyranoside hexakis sodium salt (230 mg) was obtained.

### Example 2

The product of Example 1 was converted in a known manner by hydrogenolysis into:
2-aminoethyl O-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo-α-D-glucopyranoside hexakis sodium salt. [α]_{D}²⁰ = +52° (c=0.96; water).

This product was converted by sulfation into:
2-sulfoaminoethyl O-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo-α-D-glucopyranoside heptakis sodium salt. [α]_{D}²⁰ = +55.4° (c=1.5; water).

The reaction of this product with phthalic anhydride gave:
2-(N-phthaloylamino)ethyl O-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo-α-D-glucopyranoside heptakis sodium salt. [α]_{D}²⁰ = +49.9° (c=1.23; water).

Similarly, using the corresponding anhydrides, were prepared:
2-(N-succinylamino)ethyl O-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo-α-D-glucopyranoside heptakis sodium salt. [α]_{D}²⁰ = +100° (c=0.66; water).
2-(N-trimellitylamino)ethyl O-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo-α-D-glucopyranoside heptakis sodium salt. [α]_{D}²⁰ = +47.5° (c=1.11; water).

### Example 3

### methyl O-4-O-(4-sulfoaminophenyl)-2,3,6-tri-O-sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside nonakis sodium salt.

Methyl O-4-O-(4-nitrophenyl)-6-O-acetyl-2,3-O-di-phenylmethyl-α-D-glucopyranosyl-(1→4)-O-(methyl 3-O-methyl-2-O-acetyl-α-L-idopyranosyluronate)-(1→4)-O-2,3,6-tri-O-acetyl-α-D-glucopyranoside (100 mg, 0.09 mmol), obtained by the known imidate coupling of the trichloroacetimidate of O-4-O-(4-nitrophenyl)-6-O-acetyl-2,3-O-diphenylmethyl-α-D-glucopyranoside and methyl O-(methyl 3-O-methyl-2-O-acetyl-α-L-idopyranosyluronate)-(1→4)-O- 2,3,6-tri-O-acetyl-α-D-glucopyranoside, was dissolved in tetrahydrofuran (9 ml) and cooled to -5 °C. At this temperature a 30% aq. solution of hydrogen peroxide (4.5 ml) was added to the reaction mixture, and after 10 min a 1.25 M lithium hydroxide solution (4.7 ml) was added. The mixture was stirred for 1 h at -5 °C, after which time the temperature was raised to 0 °C and the mixture was stirred overnight. The reaction mixture was acidified with 6N hydrogen chloride at 0 °C to pH 1.5, after which the saponified compound was extracted with ethyl acetate. The organic layers were pooled, dried over magnesium sulfate, and evaporated to give 63 mg (84%) of methyl O-4-O-(4-nitrophenyl)-2,3-O-di-phenylmethyl-α-D-glucopyranosyl-(1→4)-O-3-O-methyl-α-L-idopyranuronosyl-(1→4)-O-α-D-glucopyranoside, which was dissolved in methanol (8 ml). 10% Pd on charcoal (63 mg) was added and the mixture hydrogenolyzed overnight. After filtration and evaporation 27 mg (50%) of methyl O-4-O-(4-aminophenyl)-α-D-glucopyranosyl-(1→4)-O-3-O-methyl-α-L-idopyranuronosyl-(1→4)-O-α-D-glucopyranoside were obtained.
13 mg of methyl O-4-O-(4-aminophenyl)-O-α-D-glucopyranosyl-(1→4)-O-3-O-methyl-α-L-idopyranuronosyl-(1→4)-O-α-D-glucopyranoside were dissolved in 2 ml of dry N,N-dimethylformamide, and under an atmosphere of nitrogen 148 mg of triethylamine sulfurtrioxide complex were added. The mixture was stirred overnight at 50 °C, after which an aq. solution of sodium hydrogen carbonate was added under ice cooling. The mixture was stirred for 1 h at room temperature, concentrated to a small volume and desalted on a Sephadex G-10 column with water. The crude product obtained was purified by HPLC using a Mono-Q anion exchange column to give 11 mg (37%) of methyl O-4-O-(4-sulfoaminophenyl)-2,3,6-tri-O-sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside nonakis sodium salt. [α]_{D}²⁰ = +52.2° (c=0.67; water). Anomeri protons chemical shifts: 5.5; 5.17; and 5.15 ppm.

### Example 4

In a similar way as described in the previous example were prepared:
methyl O-3,4-di-O-methyl-2,6-di-O-sulfo-α-D-glucopyranosyl-(1→4)-O-2,3-di-O-methyl-α-L-idopyranuronosyl-(1→4)-O-2,3,6-tri-O-sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside undecakis sodium salt. [α]_{D}²⁰ = +26.5° (c=0.46; water). Anomeric protons chemical shifts: 5.56; 5.39; 5.31; 5.14; and 5.13 ppm.
methyl O-2,3,4-tri-O-methyl-6-O-sulfo-α-D-glucopyranosyl-(1→4)-O-2,3-di-O-methyl-β-D-glucopyranuronosyl-(1→4)-O-2,3,6-tri-O-sulfo-α-D-glucopyranosyl-(1→4)-O-2,3-di-O-methyl-α-L-idopyranuronosyl-(1→4)-O-2,3,6-tri-O-sulfo-α-D-glucopyranoside nonakis sodium salt. [α]_{D}²⁰ = +55° (c=1; water). Anomeric protons chemical shifts: 5.47; 5.42; 5.17; 5.14; and 4.67 ppm.
methyl O-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo-α-D-glucopyranosyl-(1→4)-O-2,3-di-O-methyl-α-L-idopyranuronosyl-(1→4)-O-2,3,6-tri-O-sulfo-α-D-glucopyranoside decakis sodium salt. [α]_{D}²⁰ = +53° (c=1; water). Anomeric protons chemical shifts: 5.56; 5.45; 5.19; 4.81; and 4.70 ppm.
methyl O-4-O-methyl-2,3,6-tri-O-sulfo-α-D-glucopyranosyl-(1→4)-O-2,3-di-O-methyl-β-D-glucopyranuronosyl-(1→4)-O-2,3,6-tri-O-sulfo-α-D-glucopyranosyl-(1→4)-O-2,3-di-O-methyl-α-L-idopyranuronosyl-(1→4)-O-2,3,6-tri- O-sulfo-α-D-glucopyranoside undecakis sodium salt. [α]_{D}²⁰ = +47.5° (c=1; water). Anomeric protons chemical shifts: 5.60; 5.42; 5.16; 5.09; and 4.66 ppm.
methyl O-2,3,4-tri-O-methyl-6-O-sulfo-α-D-glucopyranosyl-(1→4)-O-2,3-di-O-methyl-α-L-idopyranuronosyl-(1→4)-O-2,3,6-tri-O-sulfo-α-D-glucopyranosyl-(1→4)-O-2,3-O-di-methyl-α-L-idopyranuronosyl-(1→4)-O-2,3,6-tri-O-sulfo-α-D-glucopyranoside nonakis sodium salt. [α]_{D}²⁰ = +46.2° (c=1; water). Anomeric protons chemical shifts: 5.43; 5.37; 5.16; 5.09; and 5.09 ppm.
methyl O-2,3,4-tri-O-methyl-6-O-sulfo-α-D-glucopyranosyl-(1→4)-O-3-O-methyl-2-O-sulfo-β-D-glucopyranuronosyl-(1→4)-O-2,3,6-tri-O-sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside undecakis sodium salt. [α]_{D}²⁰ = +46.7° (c=0.55; water). Anomeric protons chemical shifts: 5.49; 5.48; 5.16; 5.15; and 4.76 ppm.
methyl O-4-O-methyl-2,3,6-tri-O-sulfo-α-D-glucopyranosyl-(1→4)-O-3-O-methyl-2-O-sulfo-β-D-glucopyranuronosyl-(1→4)-O-2,3,6-tri-O-sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside tridecakis sodium salt. [α]_{D}²⁰ = +42.2° (c=1; water). Anomeric protons chemical shifts: 5.61; 5.48; 5.15; 4.86; and 4.76 ppm.
methyl O-3,4-di-O-methyl-2,6-di-O-sulfo-α-D-glucopyranosyl-(1→4)-O-2,3-di-O-methyl-α-L-idopyranuronosyl-(1→4)-O-2,3,6-tri-O-sulfo-α-D-glucopyranosyl-(1→4)-O-2,3-di-O-methyl-α-L-idopyranuronosyl-(1→4)-O-2,3,6-tri-O-sulfo-α-D-glucopyranoside decakis sodium salt. [α]_{D}²⁰ = +42.2° (c=1; water).
methyl O-2-O-methyl-3,4,6-tri-O-sulfo-α-D-glucopyranosyl-(1→4)-O-3-O-methyl-2-O-sulfo-β-D-glucopyranuronosyl- (1→4)-O-2,3,6-tri-O-sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside tridecakis sodium salt.
methyl O-2,3-di-O-methyl-4,6-di-O-sulfo-α-D-glucopyranosyl-(1→4)-O-3-O-methyl-2-O-sulfo-β-D-glucopyranuronosyl-(1→4)-O-2,3,6-tri-O-sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside dodecakis sodium salt.
methyl O-2,4-di-O-methyl-3,6-di-O-sulfo-α-D-glucopyranosyl-(1→4)-O-3-O-methyl-2-O-sulfo-β-D-glucopyranuronosyl-(1→4)-O-2,3,6-tri-O-sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside dodecakis sodium salt.
methyl O-4-O-methyl-2,3,6-tri-O-sulfo-α-D-mannopyranosyl-(1→4)-O-2,3-di-O-methyl-β-D-glucopyranuronosyl-(1→4)-O-2,3,6-tri-O-sulfo-α-D-glucopyranosyl-(1→4)-O-2,3-di-O-methyl-α-L-idopyranuronosyl-(1→4)-O-2,3,6-tri-O-sulfo-α-D-glucopyranoside undecakis sodium salt.
methyl O-2,3,4-tri-O-methyl-α-D-glucopyranosyl-(1→4)-O-3-O-methyl-2-O-sulfo-α-L-idopyranuronosyl-(1→4)-O-2,3,6-tri-O-sulfo-α-D-glucopyranosyl-(1→4)-O-3-O-methyl-2-O-sulfo-α-L-idopyranuronosyl-(1→4)-O-2,3,6-tri-O-sulfo-α-D-glucopyranoside undecakis sodium salt.
2-(2-(4-fluorophenyloxy)ethoxy)ethyl O-3,4-di-O-methyl-2,6-di-O-sulfo-α-D-glucopyranosyl-(1→4)-O-2,3-di-O-methyl-β-D-glucopyranuronosyl-(1→4)-O-2,3,6-tri-O-sulfo-α-D-glucopyranosyl-(1→4)-O-3-O-(2-phenylethyl)-2-O-sulfo-α-L-idopyranuronosyl-(1→4)-O-2,3,6-tri-O-sulfo-α-D-glucopyranoside undecakis sodium salt. [α]_{D}²⁰ = +22.0° (c=0.3; water). Anomeric protons chemical shifts: 4.68; 4.93; 5.21; 5.46; 5.54 ppm.
2-(2-(4-fluorophenyloxy)ethoxy)ethyl O-4-O-(4-amino-phenyl)-2,3,6-tri-O-sulfo-α-D-glucopyranosyl-(1→4)-O-3-O-(2-phenylethyl)-2-O-sulfo-α-L-idopyranuronosyl-(1→4)-O-2,3,6-tri-O-sulfo-α-glucopyranoside octakis sodium salt. [α]_{D}²⁰ = +21.9° (c=1.2; water). Anomeric protons chemical shifts: 4.92; 5.27; 5.51 ppm.
2-(2-(4-fluorophenyloxy)ethoxy)ethyl O-4-O-[4-(phenylmethoxycarbonylamino)phenyl]-2,3,6-tri-O-sulfo-α-D-glucopyranosyl-(1→4)-O-3-O-(2-phenylethyl)-2-O-sulfo-α-L-idopyranuronosyl-(1→4)-O-2,3,6-tri-O-sulfo-α-D-glucopyranoside octakis sodium salt. [α]_{D}²⁰ = +20.5° (c=0.75; water). Anomeric protons chemical shifts: 4.93; 5.27; 5.52 ppm.
methyl O-2,3-di-O-methyl-4-O-sulfo-α-L-idopyranuronosyl-(1→4)-O-2,3,6-tri-O-sulfo-α-D-glucopyranosyl-(1→4)-O-2,3-di-O-methyl-α-L-idopyranuronosyl-(1→4)-O-2,3,6-tri-O-sulfo-α-D-glucopyranosyl-(1→4)-O-2,3-di-O-methyl-α-L-idopyranosyl-(1→4)-O-2,3,6-tri-O-sulfo-α-D-glucopyranoside tridecakis sodium salt. Anomeric protons chemical shifts: 5.40; 5.37; 5.10; 5.05; 5.05 ppm.
methyl 0-3,4-di-0-methyl-2,6-di-0-sulfo-α-D-galactopyranosyl-(1→4)-0-2,3-di-0-methyl-β-D-glucopyranuronosyl-(1→4)-0-2,3,6-tri-0-sulfo-α-D-glucopyranosyl-(1→4)-0-3-0-methyl-2-0-sulfo-α-L-idopyranuronosyl-(1→4)-0-2,3,6-tri-0-sulfo-α-D-glucopyranoside undecakis sodium salt. [α]_{D}²⁰ = +48.8° (c=1; water). Anomeric protons chemical shifts: 4.60; 5.06; 5.06; 5.44; and 5.52 ppm.

### Example 5

### propyl O-2,6-di-O-ethyl-3,4-di-O-sulfo-β-D-galactopyranosyl-(1→4)-O-3-O-ethyl-2-O-sulfo-α-L-idopyranuronosyl-(1→3)-O-2,6-di-O-ethyl-4-O-sulfo-β-D-galactopyranosyl-(1→4)-O-3-O-ethyl-2-O-sulfo-α-L-idopyranuronoside heptakis sodium salt.

propyl O-4-O-benzoyl-2,6-di-O-ethyl-β-D-galactopyranosyl-(1→4)-O-(methyl 2-O-benzoyl-3-O-ethyl-α-L-idopyranuronosyluronate)-(1→3)-O-4-benzoyl-2,6-di-O-ethyl-β-D-galactopyranosyl-(1→4)-O-(methyl-2-O-benzoyl-3-O-ethyl-α-L-idopyranuronoside uronate) (0.028 mmol) was dissolved in 2.79 ml of tetrahydrofuran. The mixture was cooled to -5 °C and 1.37 ml of a 33% hydrogen peroxide solution were added. After 10 min stirring 0.633 ml of a 1.25 M lithium hydroxide hydrate solution in water were added. After 1 h stirring the temperature was increased to 0 °C and the mixture was stirred for another 20 h. The mixture was brought to room temperature and 2.56 ml of methanol and 0.65 ml of 4 N sodium hydroxide were added. The mixture was stirred for another 20 h and acidified with diluted hydrochloric acid at 0-5 °C. The excess of hydrogen peroxide was destroyed with a 10% sodium sulfite solution in water and the mixture was evaporated to dryness. The residue was treated with 10 ml of dichloromethane-methanol (8:2), the salts were filtered, and the filtrate evaporated to dryness. The residue was purified on a Sephadex LH20 column and the pure fractions were pooled and evaporated to dryness to obtain propyl O-2,6-di-O-ethyl-β-D-galactopyranosyl-(1→4)-O-3-O-ethyl-α-L-idopyranuronosyl-(1→3)-O-2,6-di-O-ethyl-β-D-galactopyranosyl-(1→4)-O-3-O-ethyl-α-L-idopyranuronoside, which was dissolved in a mixture of 1.63 ml of dry dimethylformamide and 0.64 mmol triethylamine sulfurtrioxide complex. The mixture was stirred for 20 h at 50 °C, after which the mixture was cooled to room temperature and a mixture of 215 mg of sodium hydrogen carbonate in 2.8 ml of water were added. The mixture was stirred for 30 min and then evaporated to dryness. The residue was dissolved in water, desalted on Sephadex G-25, and the combined fractions were liophilized to give amorphous propyl O-2,6-di-O-ethyl-3,4-di-O-sulfo-β-D-galactopyranosyl-(1→4)-O-3-O-ethyl-2-O-sulfo-α-L-idopyranuronosyl-(1→3)-O-2,6-di-O-ethyl-4-O-sulfo-β-D-galactopyranosyl-(1→4)-O-3-O-ethyl-2-O-sulfo-α-L-idopyranuronoside heptakis sodium salt. [α]_{D}²⁰ = -16.0° (c=1; water).

### Example 6

In a similar manner as described in Example 5 were prepared:
propyl O-2,6-di-O-ethyl-3,4-di-O-sulfo-β-D-galactopyranosyl-(1→4)-O-3-O-ethyl-2-O-sulfo-α-L-idopyranuronosyl-(1→3)-O-2,6-di-O-ethyl-4-O-sulfo-β-D-galactopyranosyl-(1→4)-O-3-O-ethyl-2-O-sulfo-α-L-idopyranuronosyl-(1→3)-O-2,6-di-O-ethyl-4-O-sulfo-β-D-galactopyranosyl-(1→4)-O-3-O-ethyl-2-O-sulfo-α-L-idopyranuronoside decakis sodium salt. [α]_{D}²⁰ = -17.5° (c=1; water).
propyl O-2,6-di-O-ethyl-3,4-di-O-sulfo-β-D-galactopyranosyl-(1→4)-O-3-O-ethyl-2-O-sulfo-α-L-idopyranuronoside tetrakis sodium salt. [α]_{D}²⁰ = -9.7° (c=1; water).
4-methoxyphenyl O-3-O-methyl-2,4-di-O-sulfo-α-L-idopyranuronosyl-(1→3)-O-6-O-methyl-2,4-di-O-sulfo-β-D-galactopyranosyl-(1→4)-O-3-O-methyl-2-O-sulfo-α-L-idopyranuronosyl-(1→3)-O-6-O-methyl-2,4-di-O-sulfo-β-D-galactopyranosyl-(1→4)-O-3-O-methyl-2-O-sulfo-α-L-idopyranurosyl-(1→3)-O-6-O-methyl-2,4-di-O-sulfo-β-D-galactopyranoside tridecakis sodium salt. Anomeric protons chemical shift: 5.44; 5.42; 5.33; 5.21; 4.67; 4.65 ppm.
4-methoxyphenyl O-3-O-methyl-2,4-di-O-sulfo-α-L-idopyranuronosyl-(1→3)-O-6-O-methyl-2,4-di-O-sulfo-β-D-galactopyranosyl-(1→4)-O-3-O-methyl-2-O-sulfo-α-L-idopyranurosyl-(1→3)-O-6-O-methyl-2,4-di-O-sulfo-β-D-galactopyranoside nonakis sodium salt. Anomeric protons chemical shift: 5.44; 5.42; 5.22; 4.66 ppm.
4-methoxyphenyl O-3-O-methyl-2,4-di-O-sulfo-α-L-idopyranuronosyl-(1→3)-O-6-O-methyl-2,4-di-O-sulfo-β-D-galactopyranoside pentakis sodium salt. Anomeric protons chemical shift: 5.29; 5.34 ppm.

## Claims (Claims for the following Contracting State(s): AT, BE, CH, DE, DK, FR, GB, IT, LI, LU, NL, SE)

1. A sulfated glycosaminoglycan derivative or a pharmaceutically acceptable salt thereof, wherein all functional N-sulfate, N-acetate, and hydroxy groups are replaced by alkoxy, aryloxy, aralkoxy, O-sulfate, or OALK(OALK)ₙOX groups, wherein ALK is an aliphatic hydrocarbon group, n is 0-5, and X is an alkyl or aryl group, provided that at least two functional groups are not replaced by O-sulfate, with the proviso that glycosaminoglycan derivatives comprising the trisaccharide unit having the structure β-D-glucopyrauronosyl-(1→4)-O-2,3,6-tri-O-sulfo-α-D-glucopyranosyl-(1→4)-O-2-O-sulfo-α-L-idopyrauronosyl and alkylated, arylated, aralkylated, or sulfated derivatives thereof, are excluded.

2. The sulfated glycosaminoglycan derivative of claim 1, comprising the disaccharide unit having the formula I 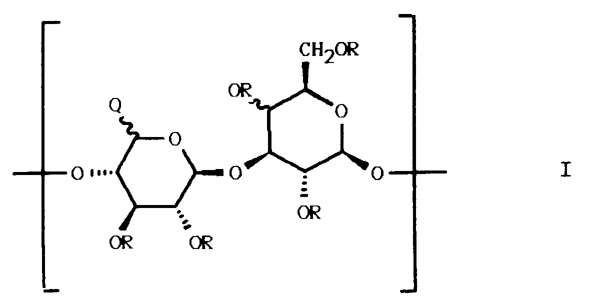 in which the twitched lines denote an α or β bond, each of the groups R are independently selected from the group consisting of alkyl, aryl, aralkyl, and sulfate; and
Q is carboxylate; or a pharmaceutically acceptable salt thereof.

3. The sulfated glycosaminoglycan derivative of claim 1, comprising the disaccharide unit having the formula IIa or IIb 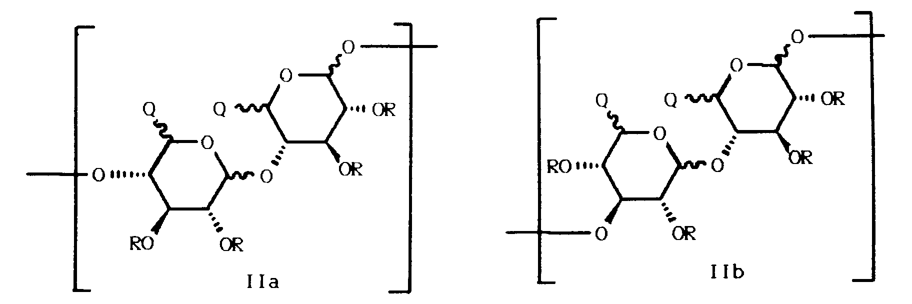 in which the twitched lines denote an α or β bond, each of the groups R are independently selected from the group consisting of alkyl, aryl, aralkyl, and sulfate, and each of the groups Q are independently selected from the group consisting of carboxylate and the CH₂OR group, or a pharmaceutically acceptable salt thereof.

4. The sulfated glycosaminoglycan derivative of claim 1, having the formula III 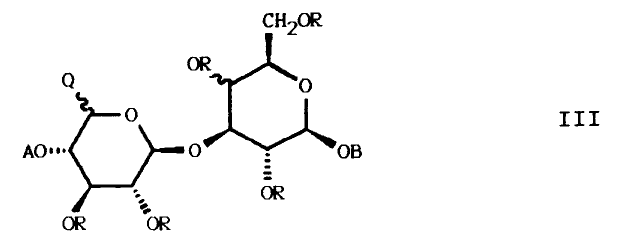 in which the twitched lines denote an α or β bond, each of the groups R are independently selected from the group consisting of alkyl, aryl, aralkyl, and sulfate;
Q is carboxylate;
A is selected from the group consisting of alkyl, aryl, aralkyl, sulfate, and a carbohydrate group having the formula IV 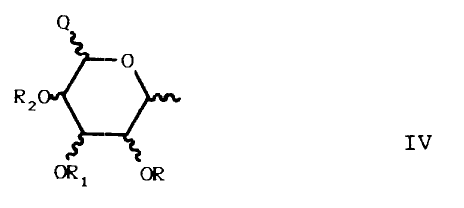 in which the twitched lines, Q, and R have the previously given meanings, one of the groups R₁ and R₂ is alkyl, aryl, aralkyl, or sulfate, whereas the other is selected from the group consisting of alkyl, aryl, aralkyl, sulfate, and carbohydrate group having the formula V 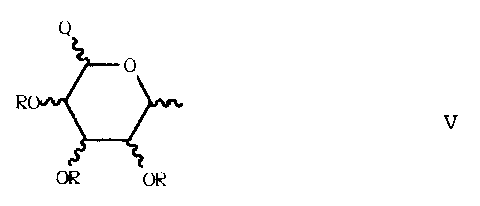 in which the twitched lines, Q, and R have the previously given meanings; and
B is selected from the group consisting of alkyl, aryl, aralkyl, sulfate, ALK(OALK)ₙOX (wherein ALK is an aliphatic hydrocarbon group, n is 0-5, and X is an alkyl or aryl group), and a carbohydrate group having the formula VI 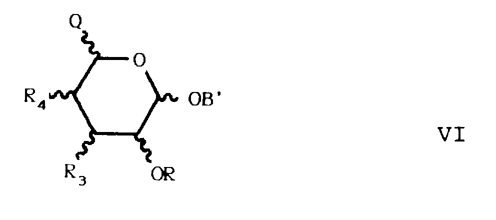 in which the twitched lines, Q, and R have the previously given meanings, one of the groups R₃ and R₄ is alkoxy, aryloxy, aralkoxy, or O-sulfate, whereas the other is a bond, and B' is selected from the group consisting of alkyl, aryl, aralkyl, sulfate, ALK(OALK)ₙOX (wherein ALK, n, and X have the previously given meanings), and a carbohydrate group having the formula VII 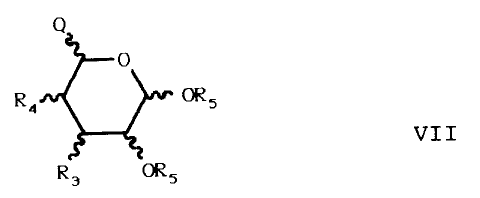 in which the twitched lines, Q, R₃, and R₄ have the previously given meanings, and each of R₅ has independently the meaning previously given for R or is ALK(OALK)ₙOX (wherein ALK, n, and X have the previously given meanings), or a pharmaceutically acceptable salt thereof.

5. The sulfated glycosaminoglycan derivative of claim 1, comprising the disaccharide unit having the formula VIII 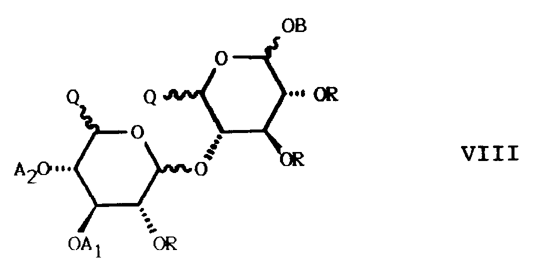 in which the twitched lines denote an α or β bond, each of the groups R are independently selected from the group consisting of alkyl, aryl, aralkyl, and sulfate, Q is carboxylate, one of the groups A₁ and A₂ is alkyl, aryl, aralkyl, or sulfate, whereas the other is selected from the group consisting of alkyl, aryl, aralkyl, sulfate, and a carbohydrate group having the formula IV 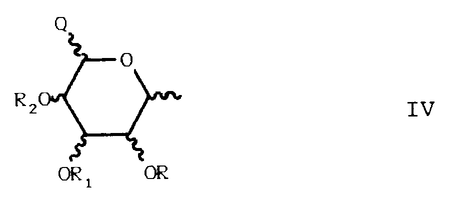 in which the twitched lines, Q, and R have the previously given meanings, one of the groups R₁ and R₂ is alkyl, aryl, aralkyl, or sulfate, whereas the other is selected from the group consisting of alkyl, aryl, aralkyl, sulfate, and a carbohydrate group having the formula V 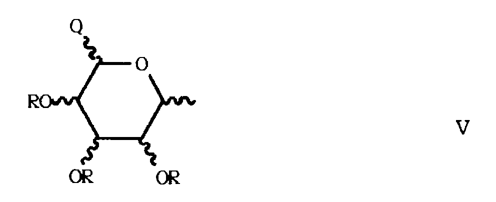 in which the twitched lines, Q, and R have the previously given meanings; and
B is selected from the group consisting of alkyl, aryl, aralkyl, sulfate, ALK(OALK)ₙOX (wherein ALK is an aliphatic hydrocarbon group, n is 0-5, and X is an alkyl or aryl group), and a carbohydrate group having the formula VI 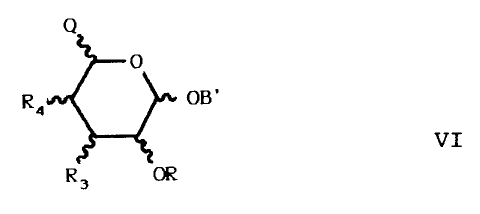 in which the twitched lines, Q, and R have the previously given meanings, one of the groups R₃ and R₄ is alkoxy, aryloxy, aralkoxy, or O-sulfate, whereas the other is a bond, and B' is selected from alkyl, aryl, aralkyl, sulfate, ALK(OALK)ₙOX, wherein ALK, n, and X have the previously given meanings, and a carbohydrate group having the formula VII 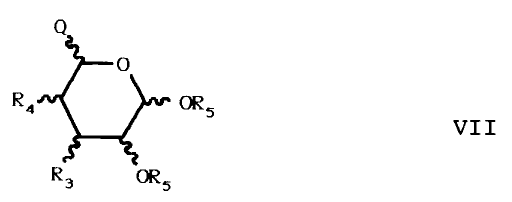 in which the twitched lines, Q, R₃, and R₄ have the previously given meanings, and each of R₅ has independently the meaning previously given for R or is ALK(OALK)ₙOX (wherein ALK, n, and X have the previously given meanings), or a pharmaceutically acceptable salt thereof.

6. The sulfated glycosaminoglycan derivative of any one of claims 1-5 for use in therapy.

7. A process for the preparation of the sulfated glycosaminoglycan derivative of any one of claims 1-5, characterized in that protected monosaccharides are coupled to give protected disaccharides, which are optionally further coupled to tri- to hexasaccharides, after which the protective groups are partially or completely cleaved and free hydroxy groups are sulfated, after which, if present, remaining protective groups are cleaved, and the compound obtained is optionally converted into a pharmaceutically acceptable salt.

8. A pharmaceutical composition comprising the sulfated glycosaminoglycan derivative of any one of claims 1-5 and pharmaceutically acceptable auxiliaries.

9. A use of the sulfated glycosaminoglycan derivative of any one of claims 1-5 for the manufacture of a medicament, having antithrombotic activity or inhibiting smooth muscle cell proliferation.

## Claims (Claims for the following Contracting State(s): ES, GR)

1. A process for the preparation of
a sulfated glycosaminoglycan derivative or a pharmaceutically acceptable salt thereof, wherein all functional N-sulfate, N-acetate, and hydroxy groups are replaced by alkoxy, aryloxy, aralkoxy, O-sulfate, or OALK(OALK)ₙOX groups, wherein ALK is an aliphatic hydrocarbon group, n is 0-5, and X is an alkyl or aryl group, provided that at least two functional groups are not replaced by O-sulfate, with the proviso that glycosaminoglycan derivatives comprising the trisaccharide unit having the structure β-D-glucopyranuronosyl-(1→4)-O-2,3,6-tri-O-sulfo-α-D-glucopyranosyl-(1→4)-O-2-O-sulfo-α-L-idopyranuronosyl and alkylated, arylated, aralkylated, or sulfated derivatives thereof, are excluded, characterized in that protected monosaccharides are coupled to give protected disaccharides, which are optionally further coupled to tri- to hexasaccharides, after which the protective groups are partially or completely cleaved and free hydroxy groups are sulfated, after which, if present, remaining protective groups are cleaved, and the compound obtained is optionally converted into a pharmaceutically acceptable salt.

2. The process of claim 1, wherein the sulfated glycosaminoglycan derivative comprises the disaccharide unit having the formula I 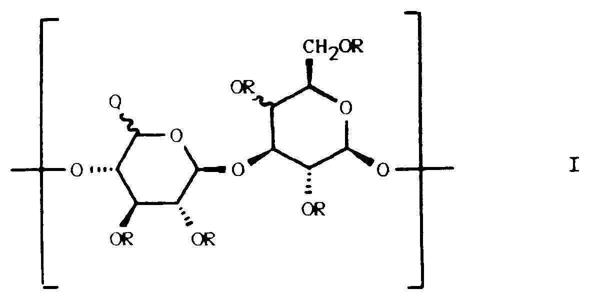 in which the twitched lines denote an α or β bond, each of the groups R are independently selected from the group consisting of alkyl, aryl, aralkyl, and sulfate; and
Q is carboxylate; or a pharmaceutically acceptable salt thereof.

3. The process of claim 1, wherein the sulfated glycosaminoglycan derivative comprises the disaccharide unit having the formula IIa or IIb 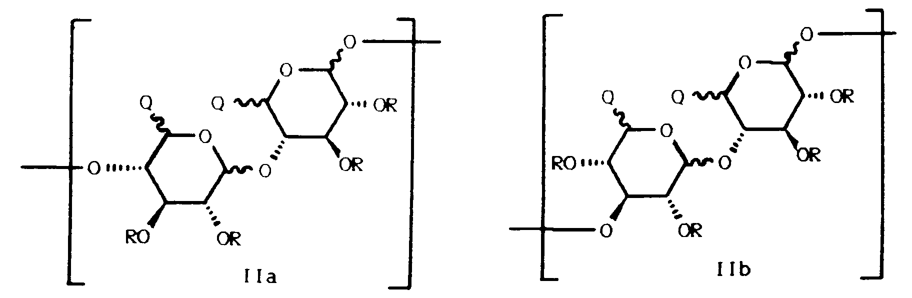 in which the twitched lines denote an α or β bond, each of the groups R are independently selected from the group consisting of alkyl, aryl, aralkyl, and sulfate, and each of the groups Q are independently selected from the group consisting of carboxylate and the CH₂OR group, or a pharmaceutically acceptable salt thereof.

4. The process of claim 1, wherein the sulfated glycosaminoglycan derivative has the formula III 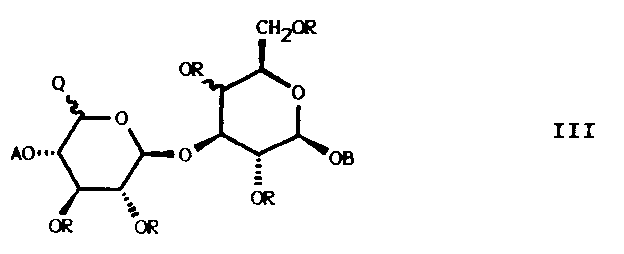 in which the twitched lines denote an α or β bond, each of the groups R are independently selected from the group consisting of alkyl, aryl, aralkyl, and sulfate;
Q is carboxylate;
A is selected from the group consisting of alkyl, aryl, aralkyl, sulfate, and a carbohydrate group having the formula IV 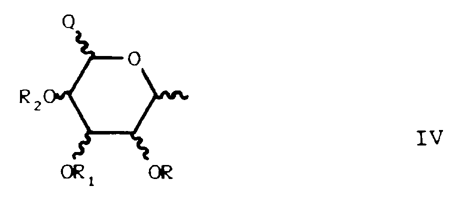 in which the twitched lines, Q, and R have the previously given meanings, one of the groups R₁ and R₂ is alkyl, aryl, aralkyl, or sulfate, whereas the other is selected from the group consisting of alkyl, aryl, aralkyl, sulfate, and carbohydrate group having the formula V 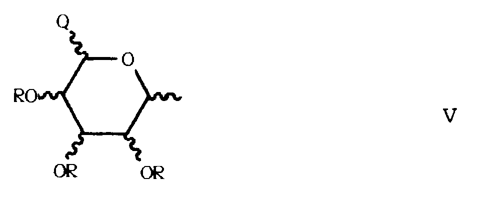 in which the twitched lines, Q, and R have the previously given meanings; and
B is selected from the group consisting of alkyl, aryl, aralkyl, sulfate, ALK(OALK)ₙOX (wherein ALK is an aliphatic hydrocarbon group, n is 0-5, and X is an alkyl or aryl group), and a carbohydrate group having the formula VI 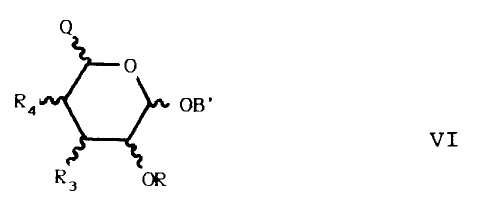 in which the twitched lines, Q, and R have the previously given meanings, one of the groups R₃ and R₄ is alkoxy, aryloxy, aralkoxy, or O-sulfate, whereas the other is a bond, and B' is selected from the group consisting of alkyl, aryl, aralkyl, sulfate, ALK(OALK)ₙOX (wherein ALK, n, and X have the previously given meanings), and a carbohydrate group having the formula VII 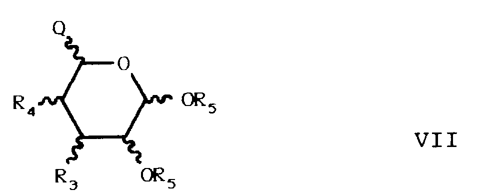 in which the twitched lines, Q, R₃, and R₄ have the previously given meanings, and each of R₅ has independently the meaning previously given for R or is ALK(OALK)ₙOX (wherein ALK, n, and X have the previously given meanings), or a pharmaceutically acceptable salt thereof.

5. The process of claim 1, wherein the sulfated glycosaminoglycan derivative comprises the disaccharide unit having the formula VIII 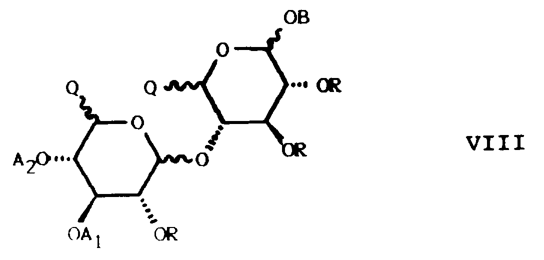 in which the twitched lines denote an α or β bond, each of the groups R are independently selected from the group consisting of alkyl, aryl, aralkyl, and sulfate, Q is carboxylate, one of the groups A₁ and A₂ is alkyl, aryl, aralkyl, or sulfate, whereas the other is selected from the group consisting of alkyl, aryl, aralkyl, sulfate, and a carbohydrate group having the formula IV 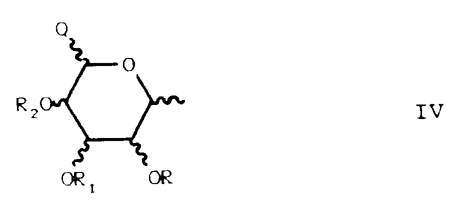 in which the twitched lines, Q, and R have the previously given meanings, one of the groups R₁ and R₂ is alkyl, aryl, aralkyl, or sulfate, whereas the other is selected from the group consisting of alkyl, aryl, aralkyl, sulfate, and a carbohydrate group having the formula V 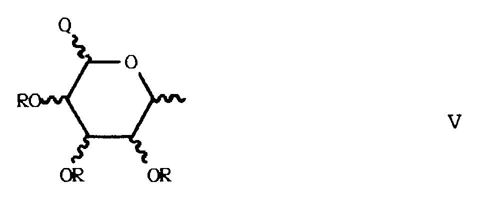 in which the twitched lines, Q, and R have the previously given meanings; and
B is selected from the group consisting of alkyl, aryl, aralkyl, sulfate, ALK(OALK)ₙOX (wherein ALK is an aliphatic hydrocarbon group, n is 0-5, and X is an alkyl or aryl group), and a carbohydrate group having the formula VI 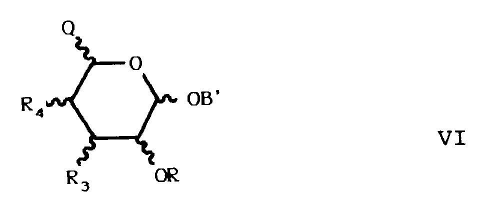 in which the twitched lines, Q, and R have the previously given meanings, one of the groups R₃ and R₄ is alkoxy, aryloxy, aralkoxy, or O-sulfate, whereas the other is a bond, and B' is selected from alkyl, aryl, aralkyl, sulfate, ALK(OALK)ₙOX, wherein ALK, n, and X have the previously given meanings, and a carbohydrate group having the formula VII 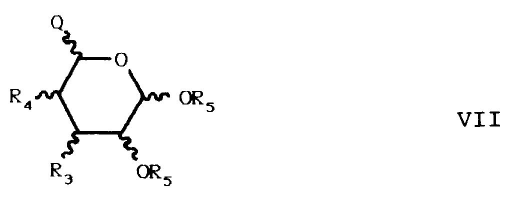 in which the twitched lines, Q, R₃, and R₄ have the previously given meanings, and each of R₅ has independently the meaning previously given for R or is ALK(OALK)ₙOX (wherein ALK, n, and X have the previously given meanings), or a pharmaceutically acceptable salt thereof.

## Claims (Claims for the following Contracting State(s): MC, PT, IE)

1. A sulfated glycosaminoglycan derivative or a pharmaceutically acceptable salt thereof, wherein all functional N-sulfate, N-acetate, and hydroxy groups, are replaced by alkoxy, aryloxy, aralkoxy, O-sulfate, or OALK(OALK)ₙOX groups, wherein ALK is an aliphatic hydrocarbon group, n is 0-5, and X is an alkyl or aryl group, provided that at least two functional groups are not replaced by O-sulfate.

2. The sulfated glycosaminoglycan derivative of claim 1, comprising the disaccharide unit having the formula I 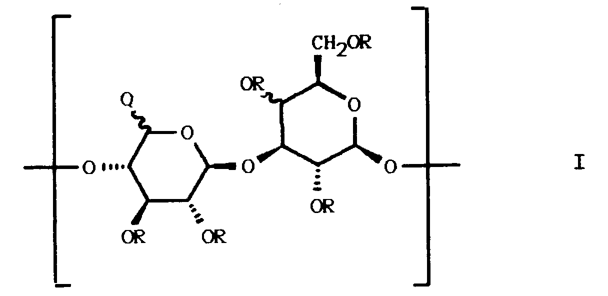 in which the twitched lines denote an α or β bond, each of the groups R are independently selected from the group consisting of alkyl, aryl, aralkyl, and sulfate; and
Q is carboxylate; or a pharmaceutically acceptable salt thereof.

3. The sulfated glycosaminoglycan derivative of claim 1, comprising the disaccharide unit having the formula IIa or IIb 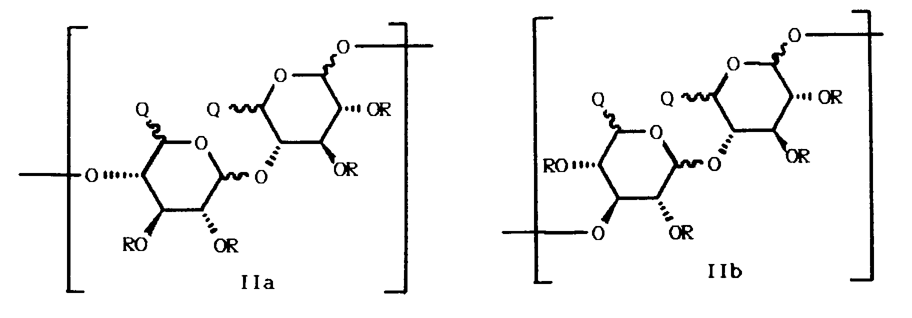 in which the twitched lines denote an α or β bond, each of the groups R are independently selected from the group consisting of alkyl, aryl, aralkyl, and sulfate, and
Q is carboxylate, or a pharmaceutically acceptable salt thereof.

4. The sulfated glycosaminoglycan derivative of claim 1, having the formula III 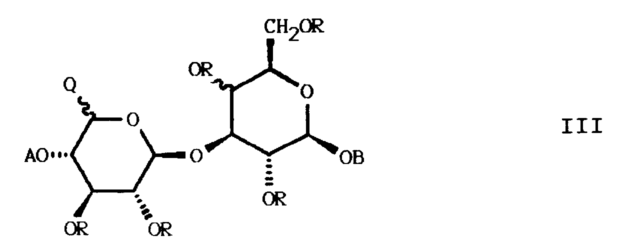 in which the twitched lines denote an α or β bond, each of the groups R are independently selected from the group consisting of alkyl, aryl, aralkyl, and sulfate;
Q is carboxylate;
A is selected from the group consisting of alkyl, aryl, aralkyl, sulfate, and a carbohydrate group having the formula IV 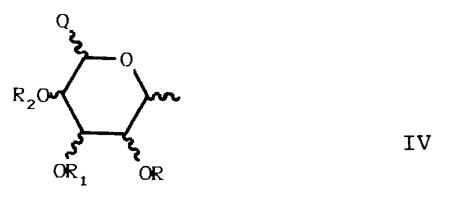 in which the twitched lines, Q, and R have the previously given meanings, one of the groups R₁ and R₂ is alkyl, aryl, aralkyl, or sulfate, whereas the other is selected from the group consisting of alkyl, aryl, aralkyl, sulfate, and a carbohydrate group having the formula V 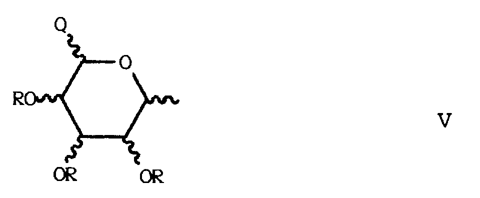 in which the twitched lines, Q, and R have the previously given meanings; and
B is selected from the group consisting of alkyl, aryl, aralkyl, sulfate, ALK(OALK)ₙOX (wherein ALK is an aliphatic hydrocarbon group, n is 0-5, and X is an alkyl or aryl group), and a carbohydrate group having the formula VI 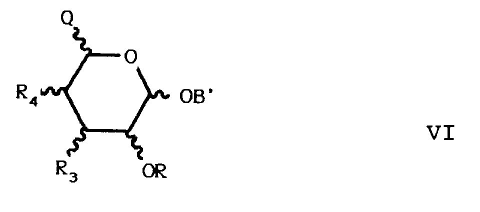 in which the twitched lines, Q, and R have the previously given meanings, one of the groups R₃ and R₄ is alkoxy, aryloxy, aralkoxy, or O-sulfate, whereas the other is a bond, and B' is selected from the group consisting of alkyl, aryl, aralkyl, sulfate, ALK(OALK)ₙOX (wherein ALK, n, and X have the previously given meanings), and a carbohydrate group having the formula VII 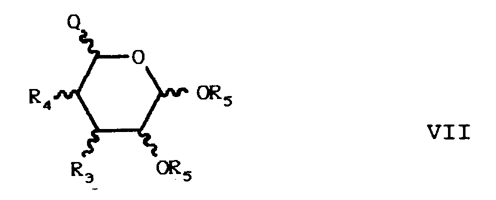 in which the twitched lines, Q, R₃, and R₄ have the previously given meanings, and each of R₅ has independently the meaning previously given for R or is ALK(OALK)ₙOX (wherein ALK, n, and X have the previously given meanings), or a pharmaceutically acceptable salt thereof.

5. The sulfated glycosaminoglycan derivative of claim 1, comprising the disaccharide unit having the formula VIII 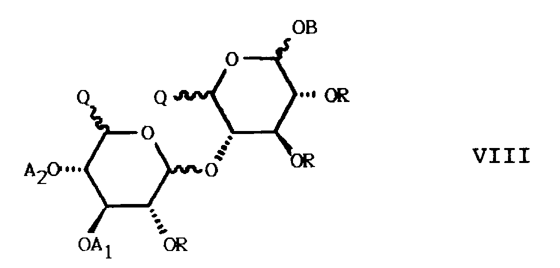 in which the twitched lines denote an α or β bond, each of the groups R are independently selected from the group consisting of alkyl, aryl, aralkyl, and sulfate, Q is carboxylate, one of the groups A₁ and A₂ is alkyl, aryl, aralkyl, or sulfate, whereas the other is selected from the group consisting of alkyl, aryl, aralkyl, sulfate, and a carbohydrate group having the formula IV 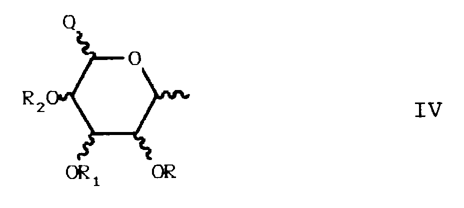 in which the twitched lines, Q, and R have the previously given meanings, one of the groups R₁ and R₂ is alkyl, aryl, aralkyl, or sulfate, whereas the other is selected from the group consisting of alkyl, aryl, aralkyl, sulfate, and a carbohydrate group having the formula V 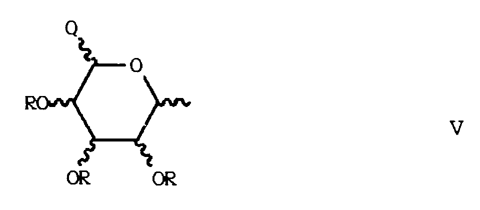 in which the twitched lines, Q, and R have the previously given meanings; and
B is selected from the group consisting of alkyl, aryl, aralkyl, sulfate, ALK(OALK)ₙOX (wherein ALK is an aliphatic hydrocarbon group, n is 0-5, and X is an alkyl or aryl group), and a carbohydrate group having the formula VI 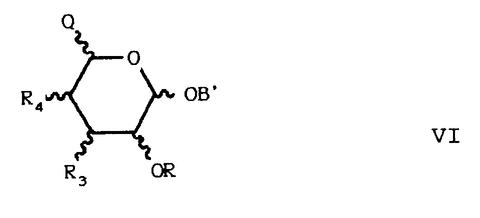 in which the twitched lines, Q, and R have the previously given meanings, one of the groups R₃ and R₄ is alkoxy, aryloxy, aralkoxy, or O-sulfate, whereas the other is a bond, and B' is selected from alkyl, aryl, aralkyl, sulfate, ALK(OALK)ₙOX, wherein ALK, n, and X have the previously given meanings, and a carbohydrate group having the formula VII 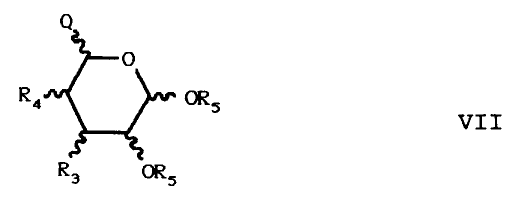 in which the twitched lines, Q, R₃, and R₄ have the previously given meanings, and each of R₅ has independently the meaning previously given for R or is ALK(OALK)ₙOX (wherein ALK, n, and X have the previously given meanings), or a pharmaceutically acceptable salt thereof.

6. The sulfated glycosaminoglycan derivative of any one of claims 1-5 for use in therapy.

7. A process for the preparation of the sulfated glycosaminoglycan derivative of any one of claims 1-5, characterized in that protected monosaccharides are coupled to give protected disaccharides, which are optionally further coupled to tri- to hexasaccharides, after which the protective groups are partially or completely cleaved and free hydroxy groups are sulfated, after which, if present, remaining protective groups are cleaved, and the compound obtained is optionally converted into a pharmaceutically acceptable salt.

8. A pharmaceutical composition comprising the sulfated glycosaminoglycan derivative of any one of claims 1-5 and pharmaceutically acceptable auxiliaries.

9. A use of the sulfated glycosaminoglycan derivative of any one of claims 1-5 for the manufacture of a medicament, having antithrombotic activity or inhibiting smooth muscle cell proliferation.

## Patentansprüche (Patentansprüche für folgende(n) Vertragsstaat(en): AT, BE, CH, DE, DK, FR, GB, IT, LI, LU, NL, SE)

1. Sulfatiertes Glucosaminoglykan-Derivat oder ein pharmazeutisch annehmbares Salz davon, dadurch gekennzeichnet, dass alle funktionellen N-Sulfat, N-Acetat- und Hydroxygruppen durch Alkoxy, Aryloxy, Aralkoxy, O-Sulfat oder OALK(OALK)ₙOX-Gruppen ersetzt sind, worin ALK eine aliphatische Kohlenwasserstoffgruppe, n 0-5 und X eine Alkyl- oder Aryl-Gruppe ist, unter der Voraussetzung, dass mindestens zwei funktionelle Gruppen nicht durch O-Sulfat ersetzt sind, unter der Bedingung, dass die Glucosaminoglykan-Derivate, die die Trisaccharid-Einheit mit der Struktur β-D-Glucopyranuronosyl-(1→4)-O-2,3,6-tri-O-sulfo-α-D-Glucopy-ranosyl-(1→4)-O-2-O-sulfo-α-L-idopyranuronosyl umfassen, und deren alkylierte, arylierte, aralkylierte oder sulfatierte Derivate ausgeschlossen sind.

2. Sulfatiertes Glucosaminoglykan-Derivat nach Anspruch 1, umfassend die Disaccharid-Einheit der Formel I, 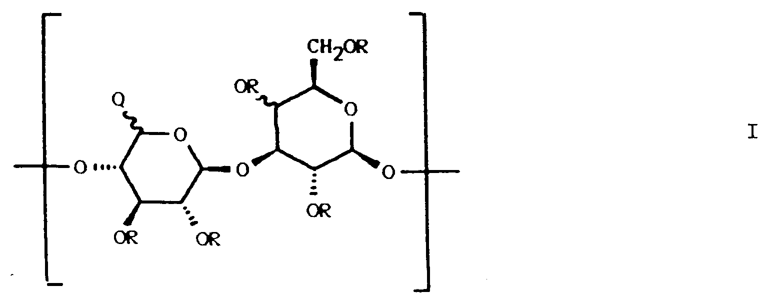 in welcher die Schlangenlinien eine α- oder β-Bindung bezeichnen, jede der R-Gruppen aus der aus Alkyl, Aryl, Aralkyl und Sulfat bestehenden Gruppe ausgewählt sind; und
Q Carboxylat ist oder ein pharmazeutisch annehmbares Salz davon.

3. Sulfatiertes Glucosaminoglykan-Derivat nach Anspruch 1, umfassend die Disaccharid-Einheit der Formel IIa oder IIb, 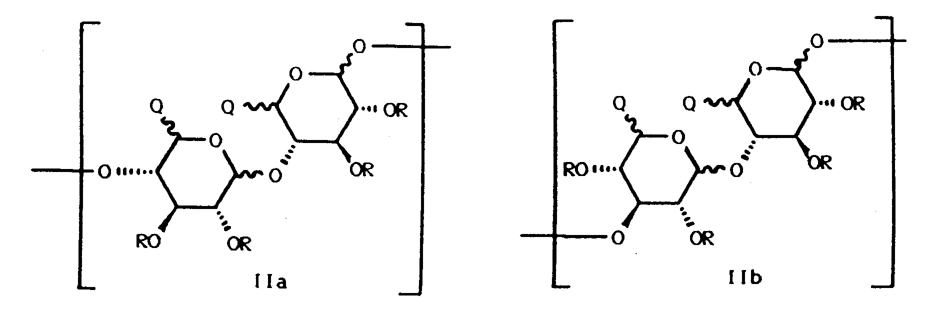 in welcher die Schlangenlinien eine α- oder β-Bindung bezeichnen, jede der R-Gruppen unabhängig aus der aus Alkyl, Aryl, Aralkyl und Sulfat bestehenden Gruppe ausgewählt ist und jede der Q-Gruppen unabhängig aus der Gruppe bestehend aus der Carboxylat- und der CH₂OR-Gruppe ausgewählt ist oder ein pharmazeutisch annehmbares Salz davon.

4. Sulfatiertes Glucosaminoglykan-Derivat nach Anspruch 1 der Formel III, 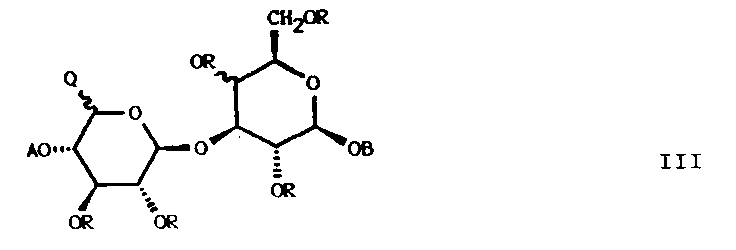 in welcher die Schlangenlinien eine α- oder β-Bindung bezeichnen, jede der R-Gruppen unabhängig aus der aus Alkyl, Aryl, Aralkyl und Sulfat bestehenden Gruppe ausgewählt ist; Q Carboxylat ist; A aus der aus Alkyl, Aryl, Aralkyl, Sulfat und einer Kohlehydratgruppe der Formel IV 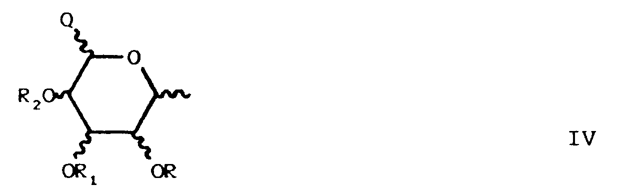 bestehenden Gruppe ausgewählt ist, in welcher Formel die Schlangenlinien, Q und R die obige Bedeutung haben, eine der Gruppen R₁ und R₂ Alkyl, Aryl, Aralkyl oder Sulfat ist, während die andere aus der aus Alkyl, Aryl, Aralkyl, Sulfat und einer Kohlehydratgruppe bestehenden Gruppe der Formel V 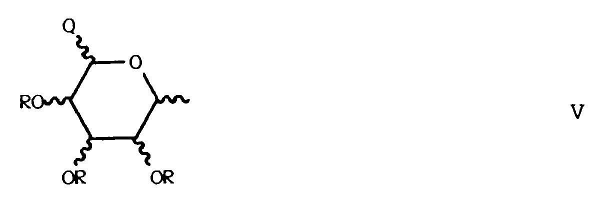 ausgewählt ist, in welcher Formel die Schlangenlinien, Q und R die obige Bedeutung haben; und
B aus der aus Alkyl, Aryl, Aralkyl, Sulfat, ALK(OALK)ₙOX (worin ALK eine aliphatische Kohlenwasserstoffgruppe, n 0-5 und X eine Alkyl- oder Aryl-Gruppe ist) und einer Kohlehydratgruppe der Formel VI 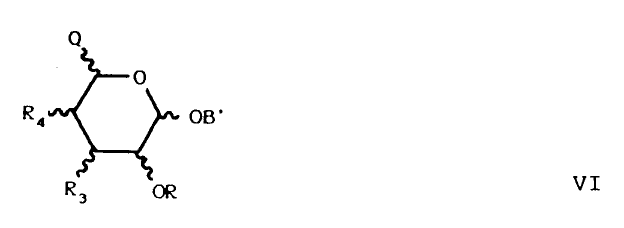 bestehenden Gruppe ausgewählt ist, in welcher Formel die Schlangenlinien, Q und R die obige Bedeutung haben, eine der Gruppen R₃ und R₄ Alkoxy, Aryloxy, Aralkoxy oder O-Sulfat ist, während die andere eine Bindung ist und B' aus der aus Alkyl, Aryl, Aralkyl, Sulfat, ALK(OALK)ₙOX (worin ALK, n und X die obige Bedeutung haben) und einer Kohlehydratgruppe der Formel VII 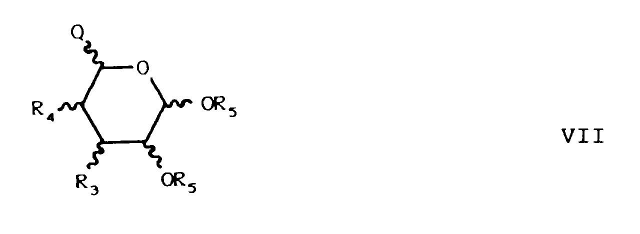 bestehenden Gruppe ausgewählt ist, in welcher Formel die Schlangenlinien, Q, R₃ und R₄ die obige Bedeutung haben und jede der R₅-Gruppen unabhängig die oben beschriebene Bedeutung von R hat oder ALK(OALK)ₙOX (worin ALK, n und X die obige Bedeutung haben) ist, oder ein pharmazeutisch annehmbares Salz davon.

5. Sulfatiertes Glucosaminoglykan-Derivat nach Anspruch 1, umfassend die Disaccharid-Einheit der Formel VIII, 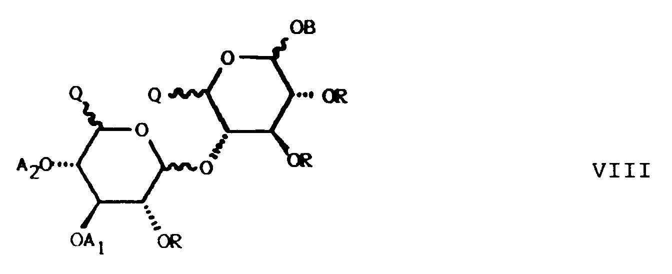 in welcher die Schlangenlinien eine α- oder β-Bindung bezeichnen, jede R-Gruppe unabhängig aus der aus Alkyl, Aryl, Aralkyl und Sulfat bestehenden Gruppe ausgewählt ist, Q Carboxylat ist, eine der Gruppen A₁ und A₂ Alkyl, Aryl, Aralkyl oder Sulfat ist, während die andere aus der aus Alkyl, Aryl, Aralkyl, Sulfat und einer Kohlehydratgruppe der Formel IV 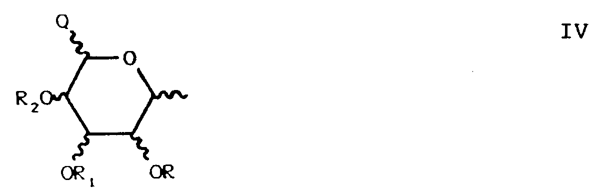 bestehenden Gruppe ausgewählt ist, in welcher Formel die Schlangenlinien, Q und R die obige Bedeutung haben, eine der Gruppen R₁ oder R₂ Alkyl, Aryl, Aralkyl oder Sulfat ist, während die andere aus der aus Alkyl, Aryl, Aralkyl, Sulfat und einer Kohlehydratgruppe der Formel V 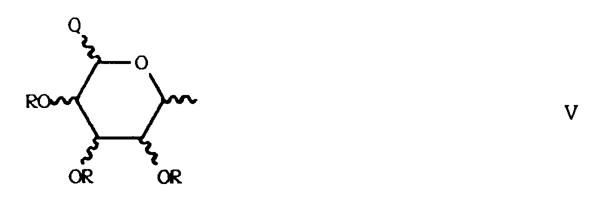 bestehenden Gruppe ausgewählt ist, in welcher Formel die Schlangenlinien, Q und R die obige Bedeutung haben; und
B aus der aus Alkyl, Aryl, Aralkyl, Sulfat, ALK(OALK)ₙOX (worin ALK eine aliphatische Kohlenwasserstoffgruppe, n 0-5 und X eine Alkyl- oder Aryl-Gruppe ist) und einer Kohlehydratgruppe der Formel VI 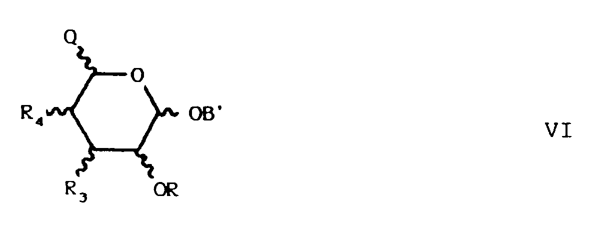 bestehenden Gruppe ausgewählt ist, in welcher Formel die Schlangenlinien, Q und R die obige Bedeutung haben, eine der Gruppen R₃ und R₄ Alkoxy, Arlyoxy, Aralkoxy oder O-Sulfat ist, während die andere eine Bindung ist und B' aus Alkyl, Aryl, Aralkyl, Sulfat, ALK(OALK)ₙOX, worin ALK, n und X die obige Bedeutung haben, und einer Kohlehydratgruppe der Formel VII 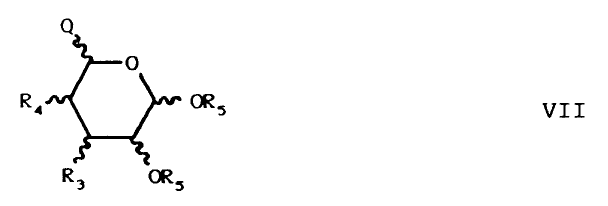 ausgewählt wird, in welcher Formel die Schlangenlinien, Q, R₃ und R₄ die obige Bedeutung haben und jede der R₅-Gruppen unabhängig die oben für R beschriebene Bedeutung haben oder ALK(OALK)ₙOX (worin ALK, n und X die obige Bedeutung haben) ist, oder ein pharmazeutisch annehmbares Salz davon.

6. Sulfatiertes Glucosaminoglykan-Derivat nach einem der Ansprüche 1 bis 5 für Therapiezwecke.

7. Verfahren zur Herstellung eines sulfatierten Glucosaminoglykan-Derivats nach einem der Ansprüche 1 bis 5, dadurch gekennzeichnet, dass geschützte Monosaccharide gekuppelt werden, um geschützte Disaccharide zu ergeben, die gegebenenfalls weiter zu Tri- bis Hexasachhariden gekuppelt werden, wonach die Schutzgruppen teilweise oder ganz abgespalten und die freien Hydroxygruppen sulfatiert werden, wonach die verbleibenden Schutzgruppen, falls vorhanden, abgespalten werden und die erhaltene Verbindung gegebenenfalls in ein pharmazeutisch annehmbares Salz umgewandelt wird.

8. Pharmazeutische Zusammensetzung, umfassend sulfatiertes Glucosaminoglykan-Derivat nach einem der Ansprüche 1 bis 5 und pharmazeutisch annehmbare Hilfsstoffe.

9. Verwendung des sulfatierten Glucosaminoglykan-Derivats nach einem der Ansprüche 1 bis 5 zur Herstellung eines Medikaments, das Antithrombose-Wirkung besitzt oder die Proliferation von glatten Muskelzellen hemmt.

## Patentansprüche (Patentansprüche für folgende(n) Vertragsstaat(en): ES, GR)

1. Verfahren zur Herstellung eines sulfatierten Glucosaminoglykan-Derivats oder eines pharmazeutisch annehmbaren Salzes davon, in welchem alle funktionellen N-Sulfate, N-Acetate und Hydroxygruppen durch Alkoxy, Aryloxy, Aralkoxy, O-Sulfat oder OALK(OALK)ₙOX-Gruppen ersetzt sind, wobei ALK eine aliphatische Kohlenwasserstoffgruppe, n 0-5 und X eine Alkyl- oder Aryl-Gruppe ist, unter der Voraussetzung, dass mindestens zwei funktionelle Gruppen nicht durch O-Sulfat ersetzt sind, unter der Bedingung, dass die Glucosaminoglykan-Derivate, die die Trisaccharid-Einheit mit der Struktur β-D-Glucopyranuronosyl-(1→4)-O-2,3,6-tri-O-sulfo-α-D-glucopyranosyl-(1→4)-O-2-O-sulfo-α-L-idopyranuronosyl umfassen, und deren alkylierte, arylierte, aralkylierte oder sulfatierte Derivate ausgeschlossen sind, dadurch gekennzeichnet, dass geschützte Monosaccharide gekuppelt werden, um geschützte Disaccharide zu erhalten, die gegebenenfalls weiter zu Tri- bis Hexasacchariden verbunden werden, wonach die Schutzgruppen teilweise oder ganz abgespalten und die freien Hydroxygruppen sulfatiert werden, wonach die verbleibenden Gruppen, falls vorhanden, abgespalten werden und die erhaltene Verbindung gegebenenfalls in ein pharmzeutisch annehmbares Salz umgewandelt wird.

2. Verfahren nach Anspruch 1, dadurch gekennzeichnet, dass das sulfatierte Glucosaminoglykan-Derivat die Disaccharid-Einheit der Formel I 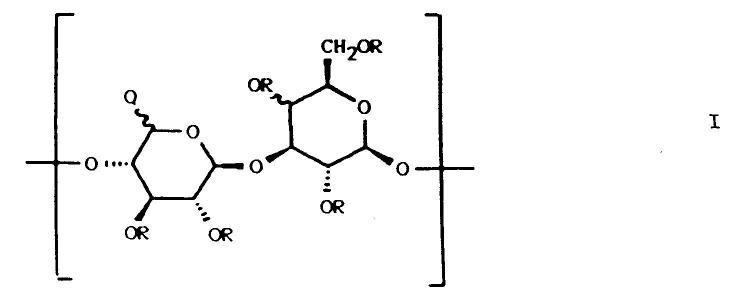 umfasst, in welcher Formel Schlangenlinien eine α- oder β-Bindung bezeichnen, jede der R-Gruppen unabhängig aus der aus Alkyl, Aryl, Aralkyl und Sulfat bestehenden Gruppe ausgewählt ist; und
Q Carboxylat ist, oder ein pharmazeutisch annehmbares Salz davon.

3. Verfahren nach Anspruch 2, dadurch gekennzeichnet, dass das sulfatierte Glucosaminoglykan-Derivat die Disaccharid-Einheit der Formel IIa oder IIb 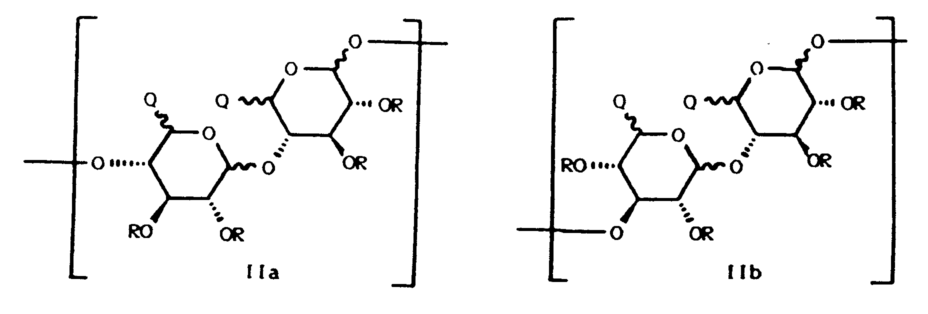 umfasst, in welcher die Schlangenlinien eine α- oder β-Bindung bezeichnen, jede der R-Gruppen unabhängig aus der aus Alkyl, Aryl, Aralkyl und Sulfat bestehenden Gruppe ausgewählt ist und jede der Q-Gruppen unabhängig aus der aus Carboxylat und der CH₂OR-Gruppe ausgewählt ist, oder ein pharmazeutisch annehmbaren Salz davon.

4. Verfahren nach Anspruch 1, dadurch gekennzeichnet, dass das sulfatierte Glucosaminoglykan-Derivat die Formel III 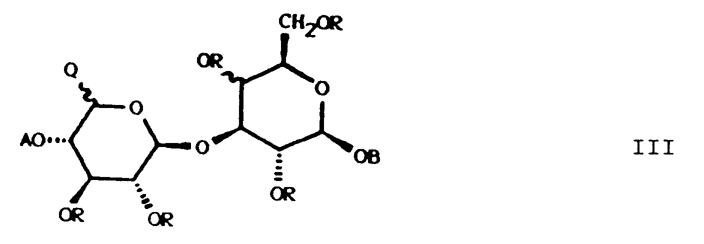 hat, in welcher die Schlangenlinien eine α- oder β-Bindung bezeichnen, jede der R-Gruppen unabhängig aus der aus Alkyl, Aryl, Aralkyl und Sulfat bestehenden Gruppe ausgewählt ist;
Q Carboxylat ist; A aus einer aus Alkyl, Aryl, Aralkyl, Sulfat und einer Kohlehydratgruppe bestehenden Gruppe der Formel IV 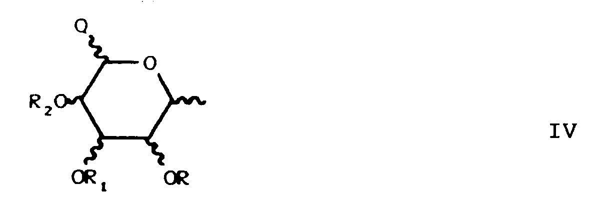 ausgewählt ist, in welcher Formel die Schlangenlinien, Q und R die obige Bedeutung haben, eine der Gruppen R₁ und R₂ Alkyl, Aryl, Aralkyl oder Sulfat ist, während die andere aus der aus Alkyl, Aryl, Aralkyl, Sulfat und einer Kohlenwasserstoffgruppe bestehenden Gruppe der Formel V 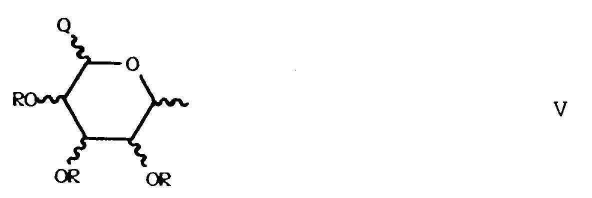 ausgewählt ist, in welcher Formel die Schlangenlinien, Q und R die obige Bedeutung haben; und
B aus der aus Alkyl, Aryl, Aralkyl, Sulfat, ALK(WALK)ₙOX (worin ALK eine aliphatische Kohlenwasserstoffgruppe, n 0-5 und X ein Alkyl- oder Aryl-Gruppe ist) und einer Kohlehydratgruppe bestehenden Gruppe der Formel VI 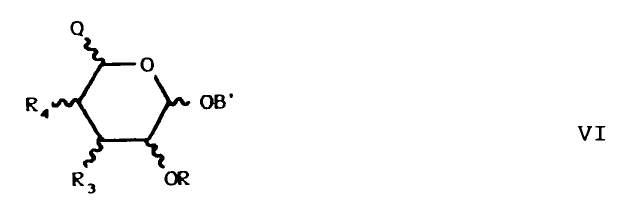 ausgewählt ist, in welcher Formel die Schlangenlinien, Q und R die obige Bedeutung haben, eine der Gruppen R₃ und R₄ Alkoxy, Aryloxy, Aralkoxy oder O-Sulfat ist, während die andere eine Bindung ist und B' aus der aus Alkyl, Aryl, Aralkyl, Sulfat, ALK(OALK)ₙOX (worin ALK, n und X die obige Bedeutung haben) und einer Kohlenwasserstoffgruppe bestehenden Gruppe der Formel VII 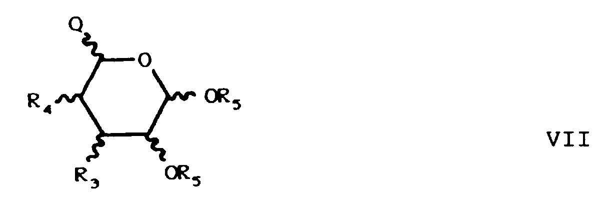 ausgewählt ist, in welcher Formel die Schlangenlinien, Q, R₃ und R₄ die obige Bedeutung haben und jede der R₅-Gruppe unabhängig die oben beschriebene Bedeutung von R hat oder ALK(OALK)ₙOX (worin ALK, n und X die obige Bedeutung haben) ist, oder ein pharmazeutisch annehmbares Salz davon.

5. Verfahren nach Anspruch 1, dadurch gekennzeichnet, dass das sulfatierte Glucosaminoglykan-Derivat die Disaccharid-Einheit der Formel VIII 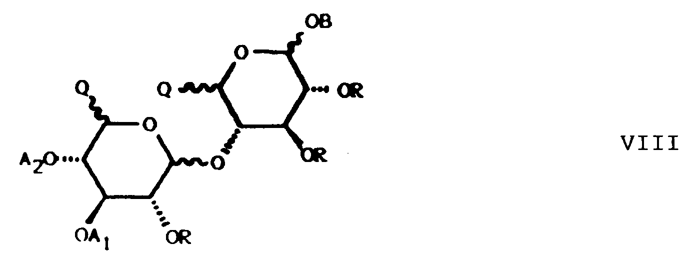 umfasst, in welcher die Schlangenlinien eine α- oder β-Bindung bezeichnen, jede R-Gruppe unabhängig aus der aus Alkyl, Aryl, Aralkyl und Sulfat bestehenden Gruppe ausgewählt ist, Q Carboxylat ist, eine der Gruppen A₁ und A₂ Alkyl, Aryl, Aralkyl oder Sulfat ist, während die andere aus der aus Alkyl, Aryl, Aralkyl, Sulfat und einer Kohlehydratgruppe bestehenden Gruppe der Formel IV 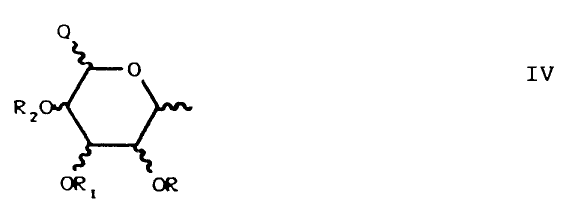 ausgewählt ist, in welcher Formel die Schlangenlinien, Q und R die obige Bedeutungn haben, eine der Gruppen R₁ oder R₂ Alkyl, Aryl, Aralkyl oder Sulfat ist, wobei die andere aus der aus Alkyl, Aryl, Aralkyl, Sulfat und einer Kohlehydratgruppe bestehenden Gruppe der Formel V 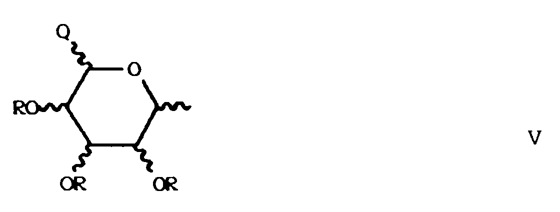 ausgewählt ist, in welcher Formel die Schlangenlinien, Q und R die obige Bedeutung haben; und
B aus der aus Alkyl, Aryl, Aralkyl, Sulfat, ALK(OALK)ₙOX (worin ALK eine aliphatische Kohlenwasserstoffgruppe, n 0-5 und X eine Alkyl- oder Aryl-Gruppe ist) und einer Kohlehydratgruppe bestehenden Gruppe der Formel VI 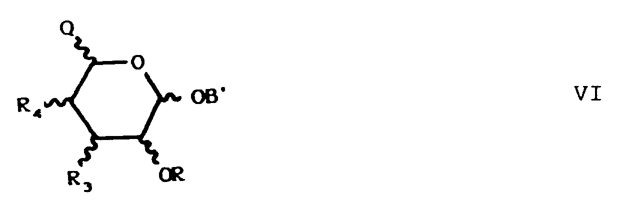 ausgewählt ist, in welcher Formel die Schlangenlinien, Q und R die obige Bedeutung haben, eine der Gruppen R₃ und R₄ Alkoxy, Arlyoxy, Aralkoxy oder O-Sulfat ist, während die andere eine Bindung ist und B' aus Alkyl, Aryl, Aralkyl, Sulfat, ALK(OALK)ₙOX, worin ALK, n und X die obige Bedeutung haben, und einer Kohlehydratgruppe der Formel VII 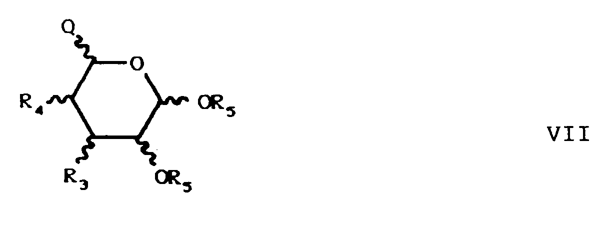 ausgewählt ist, in welcher Formel die Schlangenlinien, Q, R₃ und R₄ die obige Bedeutung haben und jede der R₅-Gruppen unabhängig die oben für R beschriebene Bedeutung hat oder ALK(OALK)ₙOX (worin ALK, n und X die obige Bedeutung haben) ist, oder ein pharmazeutisch annehmbares Salz davon.

## Patentansprüche (Patentansprüche für folgende(n) Vertragsstaat(en): MC, PT, IE)

1. Sulfatiertes Glucosaminoglykan-Derivat oder ein pharmazeutisch annehmbares Salz davon, dadurch gekennzeichnet, dass alle funktionellen N-Sulfat, N-Acetat und Hydroxygruppen durch Alkoxy, Aryloxy, Aralkoxy, O-Sulfat oder OALK(OALK)ₙOX-Gruppen, worin ALK eine aliphatische Kohlenwasserstoffgruppe, n 0-5 und X eine Alkyl- oder Arylgruppe ist, ersetzt sind, unter der Voraussetzung, dass mindestens zwei funktionelle Gruppen nicht durch O-Sulfat ersetzt sind.

2. Sulfatiertes Glucosaminoglykan-Derivat nach Anspruch 1, umfassend die Disaccharid-Einheit der Formel I, 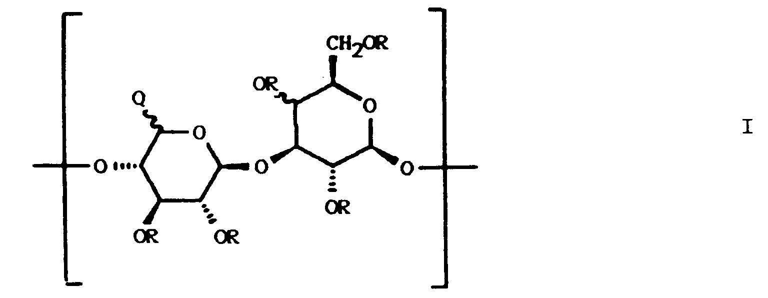 in welcher die Schlangenlinien eine α- oder β-Bindung bezeichnen, jede der R-Gruppen aus der aus Alkyl, Aryl, Aralkyl und Sulfat bestehenden Gruppe ausgewählt sind; und
Q Carboxylat ist, oder ein pharmazeutisch annehmbares Salz davon.

3. Sulfatiertes Glucosaminoglykan-Derivat nach Anspruch 1, umfassend die Disaccharid-Einheit der Formel IIa oder IIb, 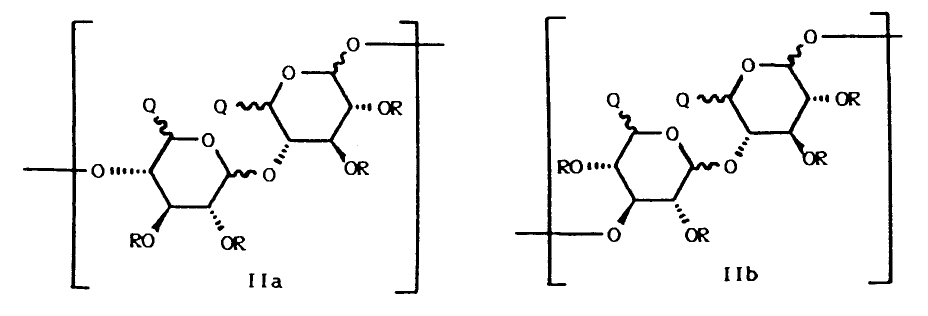 in welcher die Schlangenlinien eine α- oder β-Bindung bezeichnen, jede der R-Gruppen unabhängig aus einer aus Alkyl, Aryl, Aralkyl und Sulfat bestehenden Gruppe ausgewählt ist und Q Carboxylat, oder ein pharmazeutisch annehmbares Salz davon.

4. Sulfatiertes Glucosaminoglykan-Derivat nach Anspruch 1, der Formel III, 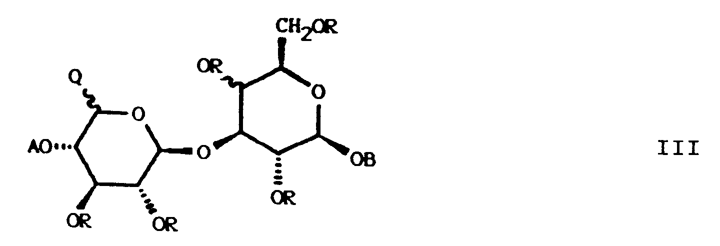 in welcher die Schlangenlinien eine α- oder β-Bindung bezeichnen, jede der R-Gruppen unabhängig aus der aus Alkyl, Aryl, Aralkyl und Sulfat bestehenden Gruppe ausgewählt ist;
Q Carboxylat ist; A aus einer aus Alkyl, Aryl, Aralkyl, Sulfat und einer Kohlehydratgruppe bestehenden Gruppe der Formel IV 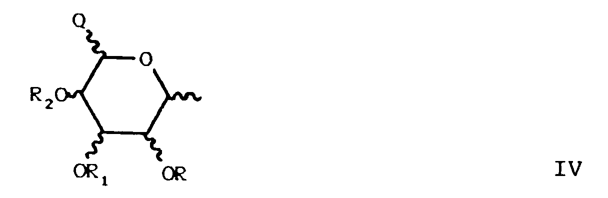 ausgewählt ist, in welcher Formel die Schlangenlinien, Q und R die obige Bedeutung haben, eine der Gruppen R₁ und R₂ Alkyl, Aryl, Aralkyl oder Sulfat ist, während die andere aus der aus Alkyl, Aryl, Aralkyl, Sulfat und einer Kohlehydratgruppe bestehenden Gruppe der Formel V 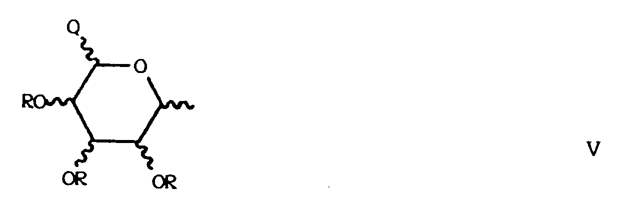 ausgewählt ist, in welcher Formel die Schlangenlinien, Q und R die obige Bedeutung haben; und
B aus der aus Alkyl, Aryl, Aralkyl, Sulfat, ALK(WALK)ₙOX (worin ALK eine aliphatische Kohlenwasserstoffgruppe, n 0-5 und X ein Alkyl- oder Aryl-Gruppe ist) und einer Kohlehydratgruppe bestehenden Gruppe der Formel VI 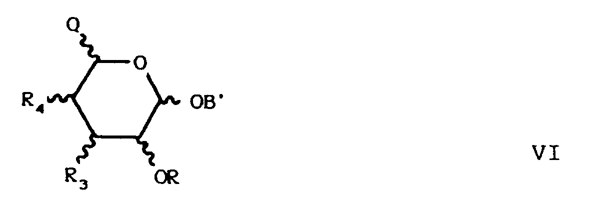 ausgewählt ist, in welcher Formel die Schlangenlinien, Q und R die obige Bedeutung haben, eine der Gruppen R₃ und R₄ Alkoxy, Aryloxy, Aralkoxy oder O-Sulfat ist, während die andere eine Bindung ist und B' aus der aus Alkyl, Aryl, Aralkyl, Sulfat, ALK(OALK)ₙOX (worin ALK, n und X die obige Bedeutung haben) und einer Kohlehydratgruppe bestehenden Gruppe der Formel VII 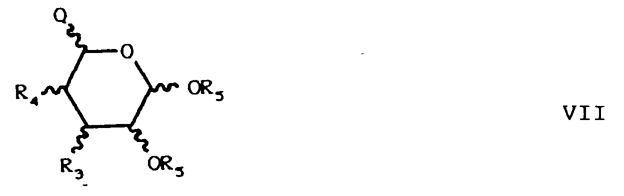 ausgewählt ist, in welcher Formel die Schlangenlinien, Q, R₃ und R₄ die obige Bedeutung haben und jede der R₅-Gruppen unabhängig die oben beschriebene Bedeutung von R hat oder ALK(OALK)ₙOX (worin ALK, n und X die obige Bedeutung haben) ist, oder ein pharmazeutisch annehmbares Salz davon.

5. Sulfatiertes Glucosaminoglykan-Derivat nach Anspruch 1, umfassend die Disaccharid-Einheit der Formel VIII, 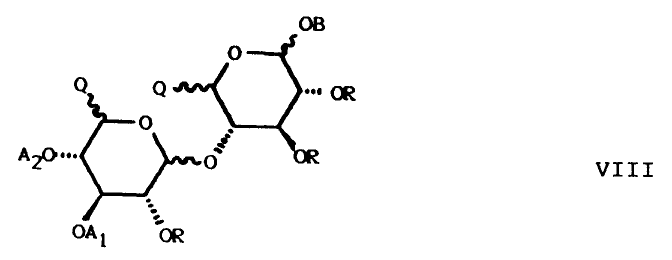 in welcher die Schlangenlinien eine α- oder β-Bindung bezeichnen, jede R-Gruppe unabhängig aus der aus Alkyl, Aryl, Aralkyl und Sulfat bestehenden Gruppe ausgewählt ist, Q Carboxylat ist, eine der Gruppen A₁ und A₂ Alkyl, Aryl, Aralkyl oder Sulfat ist, während die andere aus der aus Alkyl, Aryl, Aralkyl, Sulfat und einer Kohlehydratgruppe bestehenden Gruppe der Formel IV 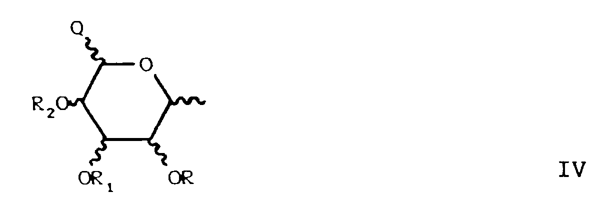 ausgewählt ist, in welcher Formel die Schlangenlinien, Q und R die obige Bedeutungn haben, eine der Gruppen R₁ oder R₂ Alkyl, Aryl, Aralkyl oder Sulfat ist, während die andere aus der aus Alkyl, Aryl, Aralkyl, Sulfat und einer Kohlehydratgruppe bestehenden Gruppe der Formel V 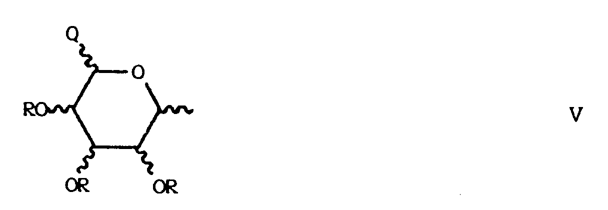 ausgewählt ist, in welcher Formel die Schlangenlinien, Q und R die obige Bedeutung haben; und
B aus der aus Alkyl, Aryl, Aralkyl, Sulfat, ALK(OALK)ₙOX (worin ALK eine aliphatische Kohlenwasserstoffgruppe, n 0-5 und X eine Alkyl- oder Aryl-Gruppe ist) und einer Kohlehydratgruppe bestehenden Gruppe der Formel VI 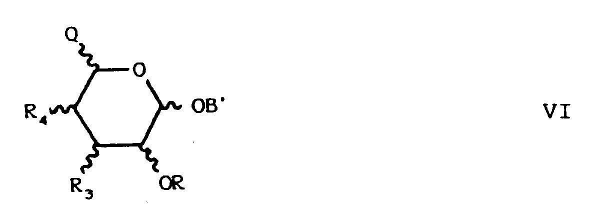 ausgewählt ist, in welcher Formel die Schlangenlinien, Q und R die obige Bedeutung haben, eine der Gruppen R₃ und R₄ Alkoxy, Arlyoxy, Aralkoxy oder O-Sulfat ist, während die andere eine Bindung ist und B' aus Alkyl, Aryl, Aralkyl, Sulfat, ALK(OALK)ₙOX, worin ALK, n und X die obige Bedeutung haben, und einer Kohlehydratgruppe der Formel VII 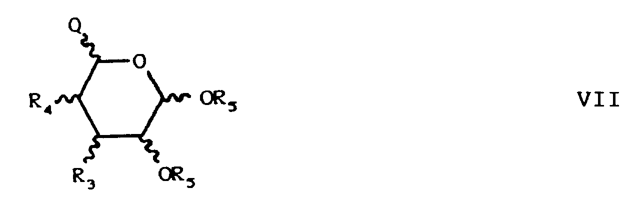 ausgewählt wird, in welcher Formel die Schlangenlinien, Q, R₃ und R₄ die obige Bedeutung haben und jede der R₅-Gruppen unabhängig die oben für R beschriebene Bedeutung haben oder ALK(OALK)ₙOX (worin ALK, n und X die obige Bedeutung haben) ist, oder ein pharmazeutisch annehmbares Salz davon.

6. Sulfatiertes Glucosaminoglykan-Derivat nach einem der Ansprüche 1 bis 5 für Therapiezwecke.

7. Verfahren zur Herstellung eines sulfatierten Glucosaminoglykan-Derivats nach einem der Ansprüche 1 bis 5, dadurch gekennzeichnet, dass geschützte Monosaccharide gekuppelt werden, um geschützte Disaccharide zu ergeben, die gegebenenfalls weiter zu Tri- bis Hexasachhariden gekuppelt werden, wonach die Schutzgruppen teilweise oder ganz abgespalten und die freien Hydroxygruppen sulfatiert werden, wonach die verbleibenden Schutzgruppen (falls vorhanden) abgespalten werden und die erhaltene Verbindung gegebenenfalls in ein pharmazeutisch annehmbares Salz umgewandelt wird.

8. Pharmazeutische Zusammensetzung, umfassend sulfatiertes Glucosaminoglykan-Derivat nach einem der Ansprüche 1 bis 5 und pharmazeutisch annehmbare Hilfsstoffe.

9. Verwendung des sulfatierten Glucosaminoglykan-Derivats nach einem der Ansprüche 1 bis 5 zur Herstellung eines Medikaments, das Antithrombose-Wirkung besitzt oder die Proliferation von glatten Muskelzellen hemmt.

## Revendications (Revendications pour l'(les) Etat(s) contractant(s) suivant(s): AT, BE, CH, DE, DK, FR, GB, IT, LI, LU, NL, SE)

1. Un dérivé sulfaté de glycosaminoglycane ou un de ses sels pharmaceutiquement acceptables, dans lequel tous les groupes fonctionnels N-sulfate, N-acétate et hydroxy sont remplacés par des groupes alcoxy, aryloxy, aralcoxy, O-sulfate, ou OALK(OALK)ₙOX, dans lequel ALK est un groupe hydrocarboné aliphatique, n est un nombre de 0 à 5, et X est un groupe alkyle ou aryle, à condition qu'au moins deux groupes fonctionnels ne soient pas remplacés par un O-sulfate, et avec cette condition que les dérivés de glycosaminoglycane comprenant le motif trisaccharide répondant à la formule β-D-glucopyranuronosyl-(1→4)-O-2,3,6,-tri-O-sulfo-α-D-glucopyranosyl-(1→4)-O-2-O-sulfo-α-L-idopyranuronosyle et leurs dérivés alkylés, arylés, aralkylés ou sulfatés sont exclus.

2. Le dérivé de glycosaminoglycane sulfaté, selon la revendication 1, comprenant le motif disaccharide répondant à la formule I : 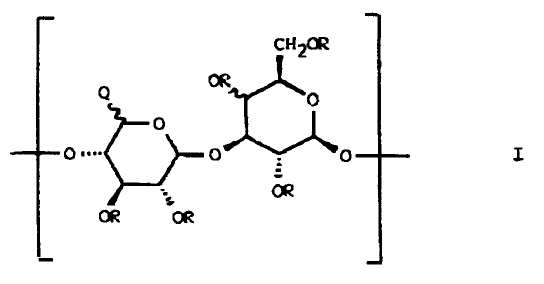 dans laquelle les lignes en pointillés désignent la liaison α ou β, chacun des groupes R étant choisi indépendamment dans le groupe consistant en alkyle, aryle, aralkyle et sulfate; et Q est un carboxylate; ou un de ses sels pharmaceutiquement acceptables.

3. Le dérivé de glycosaminoglycane sulfaté selon la revendication 1, comprenant le motif disaccharide répondant aux formules IIa ou IIb : 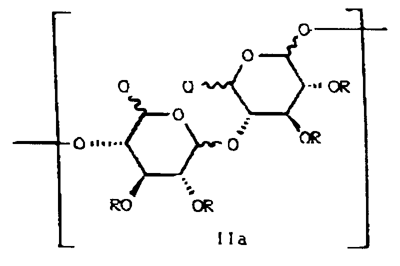 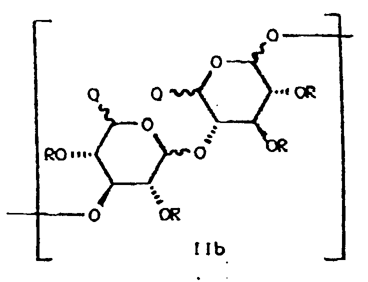 dans lesquelles les lignes en pointillés désignent une liaison α ou β, chacun des groupes R étant choisi indépendamment dans le groupe consistant en alkyle, aryle, aralkyle et sulfate, et chaque groupe Q étant choisi indépendamment dans le groupe consistant en carboxylate et groupe CH₂OR, ou un de ses sels pharmaceutiquement acceptables.

4. Le dérivé de glycosaminoglycane sulfaté selon la revendication 1, répondant à la formule III : 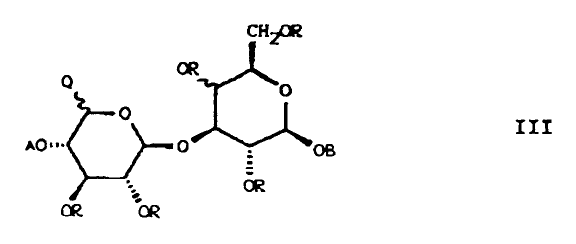 dans laquelle les lignes en pointillés désignent une liaison α ou β, chacun des groupes R étant choisi indépendamment dans le groupe consistant en alkyle, aryle, aralkyle et sulfate; Q est un carboxylate; A est choisi dans le groupe consistant en alkyle, aryle, aralkyle et sulfate, et un groupe hydrate de carbone répondant à la formule IV : 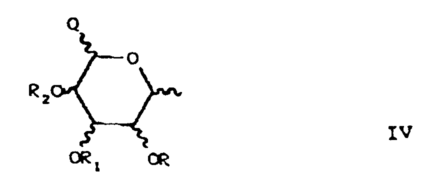 dans laquelle les lignes en pointillés, Q et R, ont les significations données ci-dessus, l'un des groupes R₁ et R₂ étant un groupe alkyle, aryle, aralkyle ou sulfate, tandis que l'autre est choisi dans le groupe consistant en alkyle, aryle, aralkyle, sulfate et un groupe hydrate de carbone répondant à la formule V : 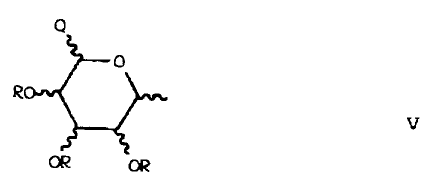 dans laquelle les lignes en pointillés, Q et R ont la signification donnée ci-dessus; et B est choisi dans le groupe consistant en alkyle, aryle, aralkyle, sulfate, ALK(OALK)ₙOX (dans lequel ALK est un groupe hydrocarboné aliphatique, n est un nombre de 0 à 5, et X est un groupe alkyle ou aryle) et un groupe hydrate de carbone répondant à la formule VI : 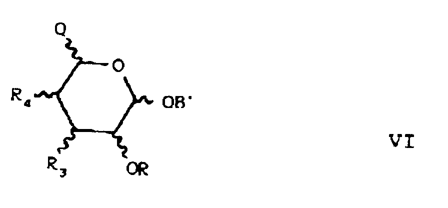 dans laquelle les lignes en pointillés, Q et R ont la signification donnée ci-dessus, l'un des groupes R₃ et R₄ est un alcoxy, aryloxy, aralcoxy, ou O-sulfate, tandis que l'autre est une liaison, et B' est choisi dans le groupe consistant en alkyle, aryle, aralkyle, sulfate, ALK(OALK)ₙOX (où ALK, n et X ont les significations données ci-dessus) et un groupe hydrate de carbone répondant à la formule VII : 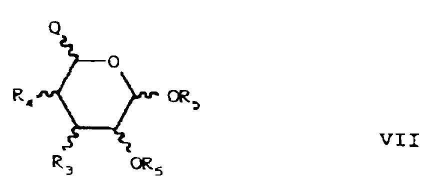 dans laquelle les lignes en pointillés Q, R₃ et R₄ ont la signification ci-dessus, chacun des R₅ a indépendamment la signification donnée ci-dessus pour R ou est un ALK(OALK)ₙOX (dans lequel ALK, n et X ont la signification donnée ci-dessus), ou un de ses sels pharmaceutiquement acceptables.

5. Le dérivé de glycosaminoglycane sulfaté selon la revendication 1, comprenant un motif disaccharide répondant à la formule VIII : 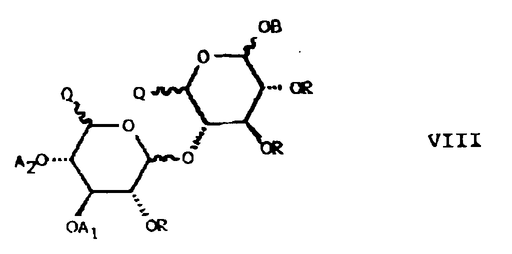 dans laquelle les lignes en pointillés désignent une liaison α ou β, chacun des groupes R étant choisi indépendamment dans le groupe consistant en alkyle, aryle, aralkyle et sulfate, Q est un carboxylate, l'un des groupes A₁ et A₂ est un alkyle, aryle, aralkyle ou sulfate, tandis que l'autre est choisi dans le groupe consistant en alkyle, aryle, aralkyle, sulfate et un groupe hydrate de carbone répondant à la formule IV : 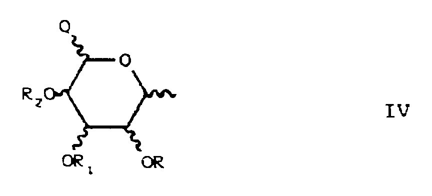 dans laquelle les lignes en pointillés, Q et R ont la signification donnée ci-dessus, l'un des groupes R₁ et R₂ est un alkyle, aryle, aralkyle ou sulfate, tandis que l'autre est choisi dans le groupe consistant en alkyle, aryle, aralkyle, sulfate et un groupe hydrate de carbone répondant à la formule V : 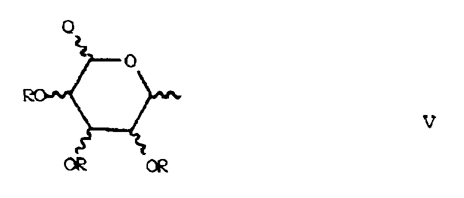 dans laquelle les lignes en pointillés, Q et R ont la signification donnée ci-dessus et
B est choisi dans le groupe consistant en alkyle, aryle, aralkyle, sulfate, ALK(OALK)ₙOX (où ALK est un groupe hydrocarboné aliphatique, n est un nombre de 0 à 5, et X est un groupe alkyle ou aryle) et un groupe hydrate de carbone répondant à la formule VI : 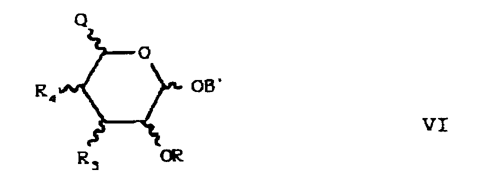 dans laquelle les lignes en pointillés, Q et R ont la signification donnée ci-dessus, l'un des groupes R₃ et R₄ est un alcoxy, aryloxy, aralcoxy ou O-sulfate, tandis que l'autre set une liaison, et B' est choisi parmi alkyle, aryle, aralkyle, sulfate, ALK(OALK)ₙOX (où ALK, n et X ont la signification donnée ci-dessus), et un groupe hydrate de carbone répondant à la formule VII : 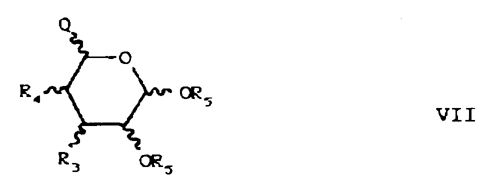 dans laquelle les lignes en pointillés, Q, R₃ et R₄ ont la signification ci-dessus, et chacun des R₅ a indépendamment la signification donnée ci-dessus pour R ou est un ALK(OALK)ₙOX (où ALK, n et X ont la signification donnée ci-dessus), ou un de ses sels pharmaceutiquement acceptables.

6. Le dérivé de glycosaminoglycane sulfaté selon l'une quelconque des revendications 1 à 5, pour son utilisation en thérapie.

7. Un procédé de préparation du dérivé de glycosaminoglycane sulfaté selon l'une quelconque des revendications 1 à 5, caractérisé en ce que l'on couple des monosaccharides protégés pour obtenir des disaccharides protégés, qui sont éventuellement couplés en tri- ou hexasaccharides, après quoi les groupes protecteurs sont partiellement ou totalement éliminés et les groupes hydroxy libres sont sulfatés, après quoi, les groupes protecteurs restants, éventuellement présents, sont éliminés et le composé obtenu est éventuellement transformé en un sel pharmaceutiquement acceptable.

8. Une composition pharmaceutique comprenant le dérivé sulfaté de glycosaminoglycane selon l'une quelconque des revendications 1 à 5, et des auxiliaires pharmaceutiquement acceptables.

9. L'utilisation du dérivé de glycosaminoglycane sulfaté selon l'une quelconque des revendications 1 à 5, pour la préparation d'un médicament présentant une activité anti-thrombotique ou d'inhibition de la prolifération des cellules de muscle lisse.

## Revendications (Revendications pour l'(les) Etat(s) contractant(s) suivant(s): ES, GR)

1. Le procédé de préparation d'un dérivé sulfaté de glycosaminoglycane ou d'un de ses sels pharmaceutiquement acceptables, dans lequel tous les groupes fonctionnels N-sulfate, N-acétate et hydroxy sont remplacés par des groupes alcoxy, aryloxy, aralcoxy, O-sulfate, ou OALK(OALK)ₙOX, dans lequel ALK est un groupe hydrocarboné aliphatique, n est un nombre de 0 à 5, et X est un groupe alkyle ou aryle, à condition qu'au moins deux groupes fonctionnels ne soient pas remplacés par un O-sulfate, et avec cette condition que les dérivés de glycosaminoglycane comprenant le motif trisaccharide répondant à la formule β-D-glucopyranuronosyl-(1→4)-O-2,3,6,-tri-O-sulfo-α-D-glucopyranosyl-(1→4)-O-2-O-sulfo-α-L-idopyranuronosyle et leurs dérivés alkylés, arylés, aralkylés ou sulfatés sont exclus, caractérisé en ce que l'on couple des monosaccharides protégés pour obtenir des disaccharides protégés, qui sont éventuellement couplés en tri- ou hexasaccharides, après quoi les groupes protecteurs sont partiellement ou totalement éliminés et les groupes hydroxy libres sont sulfatés, après quoi, les groupes protecteurs restants, éventuellement présents, sont éliminés et le composé obtenu est éventuellement transformé en un sel pharmaceutiquement acceptable.

2. Le procédé selon la revendication 1, dans lequel le dérivé de glycosaminoglycane sulfaté comprend le motif disaccharide répondant à la formule I : 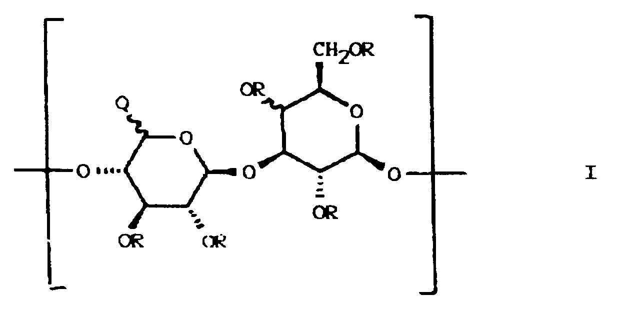 dans laquelle les lignes en pointillés désignent la liaison α ou β, chacun des groupes R étant choisi indépendamment dans le groupe consistant en alkyle, aryle, aralkyle et sulfate; et Q est un carboxylate; ou un de ses sels pharmaceutiquement acceptables.

3. Le procédé selon la revendication 1, dans lequel le dérivé de glycosaminoglycane sulfate comprend le motif disaccharide répondant aux formules IIa ou IIb : 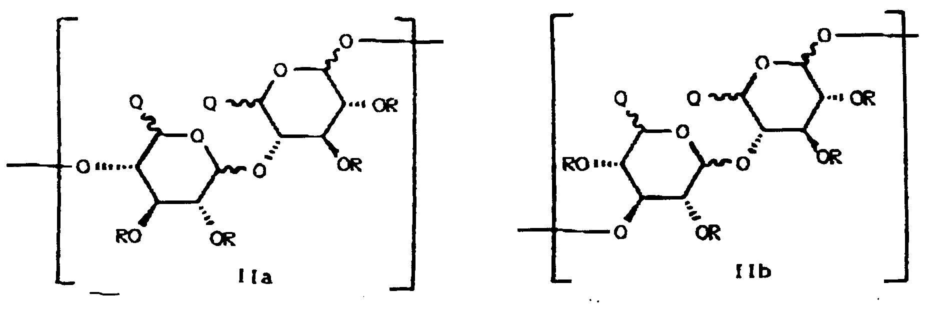 dans lesquelles les lignes en pointillés désignent une liaison α ou β, chacun des groupes R étant choisi indépendamment dans le groupe consistant en alkyle, aryle, aralkyle et sulfate, et chaque groupe Q étant choisi indépendamment dans le groupe consistant en carboxylate et groupe CH₂OR, ou un de ses sels pharmaceutiquement acceptables.

4. Le procédé selon la revendication 1, dans lequel le dérivé de glycosaminoglycane sulfaté répond à la formule III : 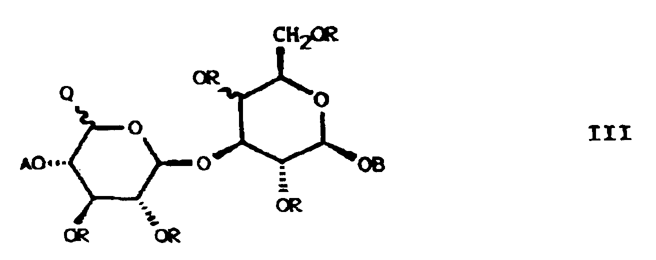 dans laquelle les lignes en pointillés désignent une liaison α ou β, chacun des groupes R étant choisi indépendamment dans le groupe consistant en alkyle, aryle, aralkyle et sulfate; Q est un carboxylate; A est choisi dans le groupe consistant en alkyle, aryle, aralkyle et sulfate, et un groupe hydrate de carbone répondant à la formule IV : 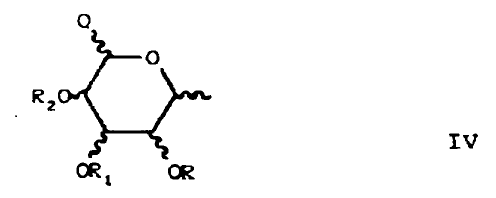 dans laquelle les lignes en pointillés, Q et R, ont les significations données ci-dessus, l'un des groupes R₁ et R₂ étant un groupe alkyle, aryle, aralkyle ou sulfate, tandis que l'autre est choisi dans le groupe consistant en alkyle, aryle, aralkyle, sulfate et un groupe hydrate de carbone répondant à la formule V : 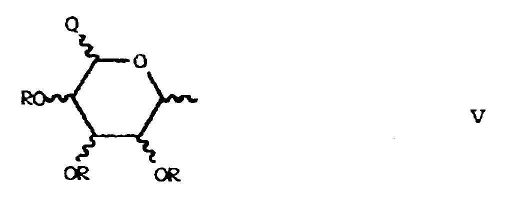 dans laquelle les lignes en pointillés, Q et R ont la signification donnée ci-dessus; et
B est choisi dans le groupe consistant en alkyle, aryle, aralkyle, sulfate, ALK(OALK)ₙOX (dans lequel ALK est un groupe hydrocarboné aliphatique, n est un nombre de 0 à 5, et X est un groupe alkyle ou aryle) et un groupe hydrate de carbone répondant à la formule VI : 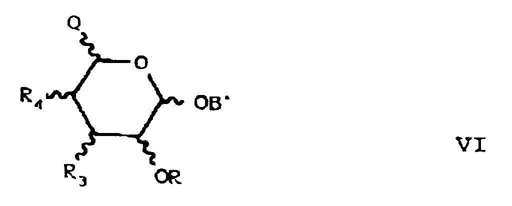 dans laquelle les lignes en pointillés, Q et R ont la signification donnée ci-dessus, l'un des groupes R₃ et R₄ est un alcoxy, aryloxy, aralcoxy, ou O-sulfate, tandis que l'autre est une liaison, et B' est choisi dans le groupe consistant en alkyle, aryle, aralkyle, sulfate, ALK(OALK)ₙOX (où ALK, n et X ont les significations données ci-dessus) et un groupe hydrate de carbone répondant à la formule VII : 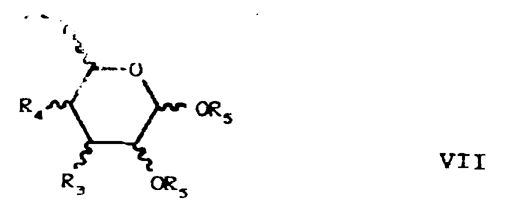 dans laquelle les lignes en pointillés Q, R₃ et R₄ ont la signification ci-dessus, chacun des R₅ a indépendamment la signification donnée ci-dessus pour R ou est un ALK(OALK)ₙOX (dans lequel ALK, n et X ont la signification donnée ci-dessus), ou un de ses sels pharmaceutiquement acceptables.

5. Le procédé selon la revendication 1, dans lequel le dérivé de glycosaminoglycane sulfaté comprend un motif disaccharide répondant à la formule VIII : 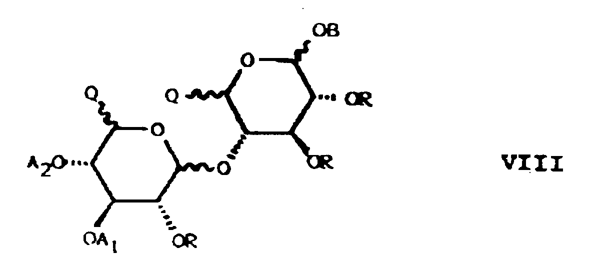 dans laquelle les lignes en pointillés désignent une liaison α ou β, chacun des groupes R étant choisi indépendamment dans le groupe consistant en alkyle, aryle, aralkyle et sulfate, Q est un carboxylate, l'un des groupes A₁ et A₂ est un alkyle, aryle, aralkyle ou sulfate, tandis que l'autre est choisi dans le groupe consistant en alkyle, aryle, aralkyle, sulfate et un groupe hydrate de carbone répondant à la formule IV : 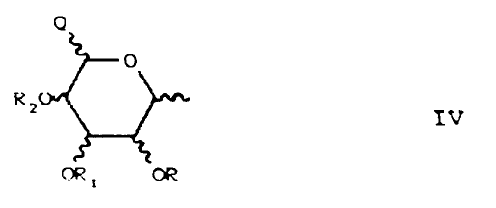 dans laquelle les lignes en pointillés, Q et R ont la signification donnée ci-dessus, l'un des groupes R₁ et R₂ est un alkyle, aryle, aralkyle ou sulfate, tandis que l'autre est choisi dans le groupe consistant en alkyle, aryle, aralkyle, sulfate et un groupe hydrate de carbone répondant à la formule V : 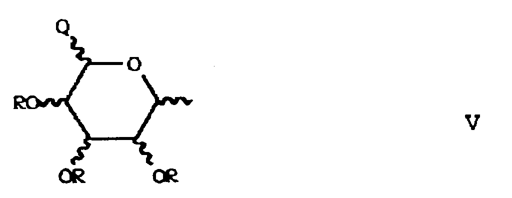 dans laquelle les lignes en pointillés, Q et R ont la signification donnée ci-dessus et B est choisi dans le groupe consistant en alkyle, aryle, aralkyle, sulfate, ALK(OALK)ₙOX (où ALK est un groupe hydrocarboné aliphatique, n est un nombre de 0 à 5, et X est un groupe alkyle ou aryle) et un groupe hydrate de carbone répondant à la formule VI : 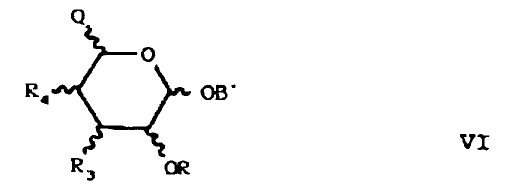 dans laquelle les lignes en pointillés, Q et R ont la signification donnée ci-dessus, l'un des groupes R₃ et R₄ est un alcoxy, aryloxy, aralcoxy ou O-sulfate, tandis que l'autre set une liaison, et B' est choisi parmi alkyle, aryle, aralkyle, sulfate, ALK(OALK)ₙOX (où ALK, n et X ont la signification donnée ci-dessus), et un groupe hydrate de carbone répondant à la formule VII : 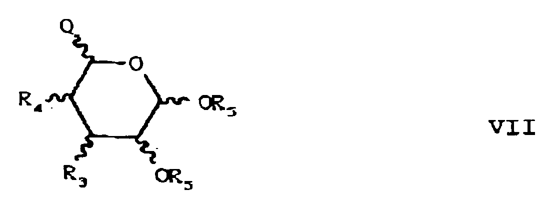 dans laquelle les lignes en pointillés, Q, R₃ et R₄ ont la signification ci-dessus, et chacun des R₅ a indépendamment la signification donnée ci-dessus pour R ou est un ALK(OALK)ₙOX (où ALK, n et X ont la signification donnée ci-dessus), ou un de ses sels pharmaceutiquement acceptables.

## Revendications (Revendications pour l'(les) Etat(s) contractant(s) suivant(s): MC, PT, IE)

1. Un dérivé sulfaté de glycosaminoglycane ou un de ses sels pharmaceutiquement acceptables, dans lequel tous les groupes fonctionnels N-sulfate, N-acétate et hydroxy sont remplacés par des groupes alcoxy, aryloxy, aralcoxy, O-sulfate, ou OALK(OALK)ₙOX, dans lequel ALK est un groupe hydrocarboné aliphatique, n est un nombre de 0 à 5, et X est un groupe alkyle ou aryle, à condition qu'au moins deux groupes fonctionnels ne soient pas remplacés par un O-sulfate.

2. Le dérivé de glycosaminoglycane sulfaté, selon la revendication 1, comprenant le motif disaccharide répondant à la formule I : 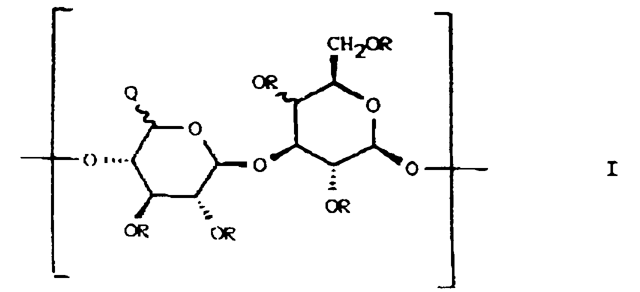 dans laquelle les lignes en pointillés désignent la liaison α ou β, chacun des groupes R étant choisi indépendamment dans le groupe consistant en alkyle, aryle, aralkyle et sulfate; et Q est un carboxylate; ou un de ses sels pharmaceutiquement acceptables.

3. Le dérivé de glycosaminoglycane sulfaté selon la revendication 1, comprenant le motif disaccharide répondant aux formules IIa ou IIb : 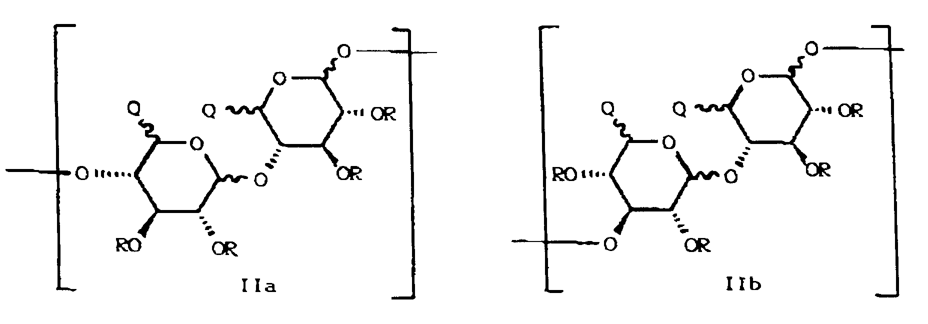 dans lesquelles les lignes en pointillés désignent une liaison α ou β, chacun des groupes R étant choisi indépendamment dans le groupe consistant en alkyle, aryle, aralkyle et sulfate, et Q est un carboxylate ou un de ses sels pharmaceutiquement acceptables.

4. Le dérivé de glycosaminoglycane sulfaté selon la revendication 1, répondant à la formule III : 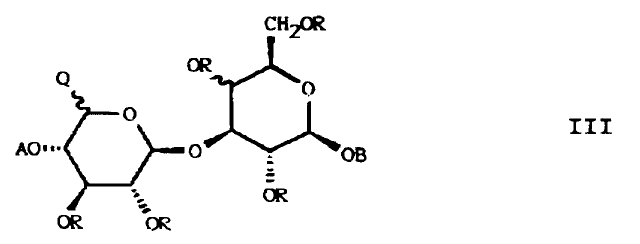 dans laquelle les lignes en pointillés désignent une liaison α ou β, chacun des groupes R étant choisi indépendamment dans le groupe consistant en alkyle, aryle, aralkyle et sulfate; Q est un carboxylate; A est choisi dans le groupe consistant en alkyle, aryle, aralkyle et sulfate, et un groupe hydrate de carbone répondant à la formule IV : 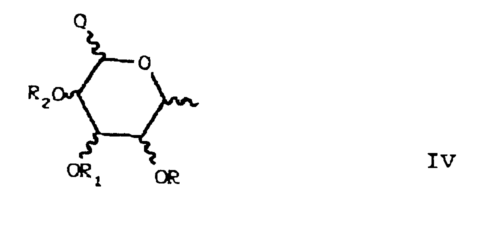 dans laquelle les lignes en pointillés, Q et R, ont les significations données ci-dessus, l'un des groupes R₁ et R₂ étant un groupe alkyle, aryle, aralkyle ou sulfate, tandis que l'autre est choisi dans le groupe consistant en alkyle, aryle, aralkyle, sulfate et un groupe hydrate de carbone répondant à la formule V : 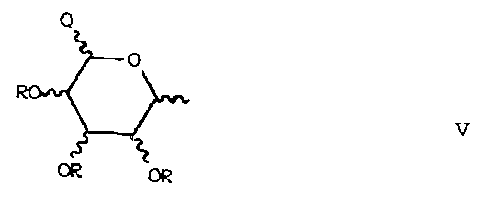 dans laquelle les lignes en pointillés, Q et R ont la signification donnée ci-dessus; et
B est choisi dans le groupe consistant en alkyle, aryle, aralkyle, sulfate, ALK(OALK)ₙOX (dans lequel ALK est un groupe hydrocarboné aliphatique, n est un nombre de 0 à 5, et X est un groupe alkyle ou aryle) et un groupe hydrate de carbone répondant à la formule VI : 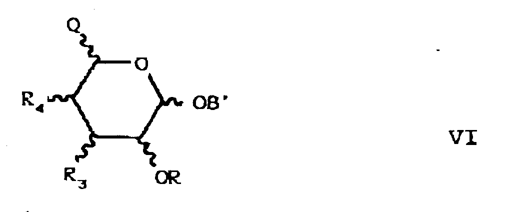 dans laquelle les lignes en pointillés, Q et R ont la signification donnée ci-dessus, l'un des groupes R₃ et R₄ est un alcoxy, aryloxy, aralcoxy, ou O-sulfate, tandis que l'autre est une liaison, et B' est choisi dans le groupe consistant en alkyle, aryle, aralkyle, sulfate, ALK(OALK)ₙOX (où ALK, n et X ont les significations données ci-dessus) et un groupe hydrate de carbone répondant à la formule VII : 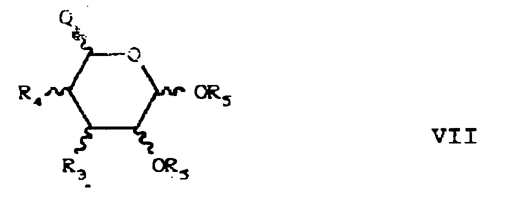 dans laquelle les lignes en pointillés Q, R₃ et R₄ ont la signification ci-dessus, chacun des R₅ a indépendamment la signification donnée ci-dessus pour R ou est un ALK(OALK)ₙOX (dans lequel ALK, n et X ont la signification donnée ci-dessus), ou un de ses sels pharmaceutiquement acceptables.

5. Le dérivé de glycosaminoglycane sulfaté selon la revendication 1, répondant à la formule VIII : 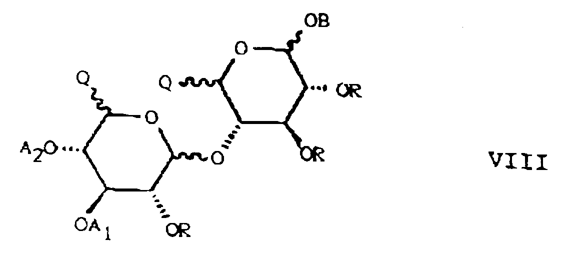 dans laquelle les lignes en pointillés désignent une liaison α ou β, chacun des groupes R étant choisi indépendamment dans le groupe consistant en alkyle, aryle, aralkyle et sulfate; Q est un carboxylate; A est choisi dans le groupe consistant en alkyle, aryle, aralkyle et sulfate, et un groupe hydrate de carbone répondant à la formule IV : 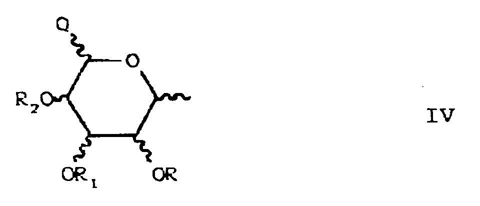 dans laquelle les lignes en pointillés, Q et R ont les significations données ci-dessus, l'un des groupes R₁ et R₂ étant un groupe alkyle, aryle, aralkyle ou sulfate, tandis que l'autre est choisi dans le groupe consistant en alkyle, aryle, aralkyle, sulfate et un groupe hydrate de carbone répondant à la formule V : 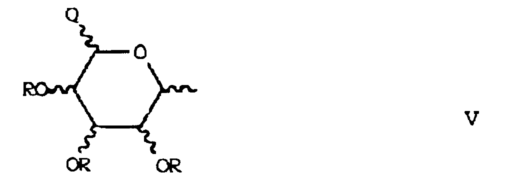 dans laquelle les lignes en pointillés, Q et R ont la signification donnée ci-dessus; et
B est choisi dans le groupe consistant en alkyle, aryle, aralkyle, sulfate, ALK(OALK)ₙOX (dans lequel ALK est un groupe hydrocarboné aliphatique, n est un nombre de 0 à 5, et X est un groupe alkyle ou aryle) et un groupe hydrate de carbone répondant à la formule VI : 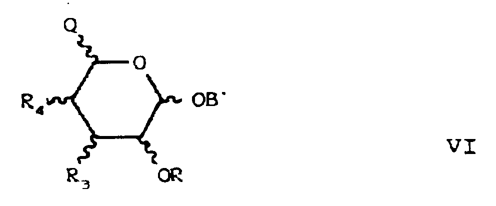 dans laquelle les lignes en pointillés, Q et R ont la signification donnée ci-dessus, l'un des groupes R₃ et R₄ est un alcoxy, aryloxy, aralcoxy, ou O-sulfate, tandis que l'autre est une liaison, et B' est choisi dans le groupe consistant en alkyle, aryle, aralkyle, sulfate, ALK(OALK)ₙOX (où ALK, n et X ont les significations données ci-dessus) et un groupe hydrate de carbone répondant à la formule VII : 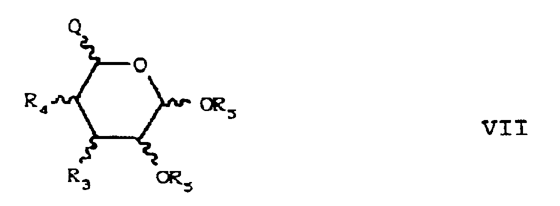 dans laquelle les lignes en pointillés Q, R₃ et R₄ ont la signification ci-dessus, chacun des R₅ a indépendamment la signification donnée ci-dessus pour R ou est un ALK(OALK)ₙOX (dans lequel ALK, n et X ont la signification donnée ci-dessus), ou un de ses sels pharmaceutiquement acceptables.

6. Le dérivé de glycosaminoglycane sulfaté selon l'une quelconque des revendications 1 à 5, pour son utilisation en thérapie.

7. Un procédé de préparation du dérivé de glycosaminoglycane sulfaté selon l'une quelconque des revendications 1 à 5, caractérisé en ce que l'on couple des monosaccharides protégés pour obtenir des disaccharides protégés, qui sont éventuellement couplés en tri- ou hexasaccharides, après quoi les groupes protecteurs sont partiellement ou totalement éliminés et les groupes hydroxy libres sont sulfatés, après quoi, les groupes protecteurs restants, éventuellement présents, sont éliminés et le composé obtenu est éventuellement transformé en un sel pharmaceutiquement acceptable.

8. Une composition pharmaceutique comprenant le dérivé sulfaté de glycosaminoglycane selon l'une quelconque des revendications 1 à 5, et des auxiliaires pharmaceutiquement acceptables.

9. L'utilisation du dérive de glycosaminoglycane sulfaté selon l'une quelconque des revendications 1 à 5, pour la préparation d'un médicament présentant une activité anti-thrombotique ou d'inhibition de la prolifération des cellules de muscle lisse.
